# EUROPEAN PATENT APPLICATION

(11) **EP 4 293 046 A1**
(43) Date of publication of application: **20.12.2023**
(21) Application number: 22752264.6
(22) Date of filing: 09.02.2022
(51) Int. Cl.: C07K 16/28, A61K 39/395, A61P 35/00

(54) **ANTI-PD-1 ANTIBODY AND USE THEREOF**

(30) Priority: 10.02.2021 CN 202110183555
(71) Applicant: Shanghai Jemincare Pharmaceuticals Co., Ltd., Pudong New Area, Shanghai 201203 (CN); Jiangxi Jemincare Group Co., Ltd., Nanchang, Jiangxi 330096 (CN)
(72) Inventor: YANG, Xinxiu, Shanghai 201203 (CN); WANG, Zongda, Shanghai 201203 (CN); LIU, Xiaowu, Shanghai 201203 (CN); GU, Chunyin, Shanghai 201203 (CN); CAO, Xiaodan, Shanghai 201203 (CN); DENG, Sujun, Shanghai 201203 (CN); PAN, Zhongzong, Shanghai 201203 (CN); WANG, Xueping, Shanghai 201203 (CN)
(74) Representative: Bianchetti & Minoja with Trevisan & Cuonzo IPS SRL
(86) International application number: PCT/CN2022/075593
(87) International publication number: WO 2022/171104

(57) **Abstract**

Provided is an isolated antigen-binding protein capable of binding to a PD-1 protein, capable of blocking binding between a PD-1 protein and a PD-L1 and/or PD-L2 protein, and/or capable of stimulating immune cells to secrete cytokines. Also provided is a use of the isolated antigen-binding protein in preparation of medicaments.

## Description

### FIELD OF THE INVENTION

The present application relates to the field of biomedicine, in particular to an anti-PD-1 antibody and its use in the manufacture of a medicament.

### BACKGROUND OF THE INVENTION

Malignant tumor is a disease that seriously threaten human health worldwide at present, and it is the main type of disease that causes human death. With the aging of population in China, the incidence of cancer is increasing, and effective therapeutic drugs need to be developed urgently, among which the development of immune checkpoint drugs has become a research hotspot in recent years.

Programmed death receptor 1 (PD-1) is widely expressed in immune cells, and it is an important immunosuppressive molecule. The main ligand of PD-1 is PD-L1, which is mainly expressed on the surface of tumor cells. After binding of the ligand PD-L1 to the receptor PD-1, the activation of T cells is inhibited in the tumor microenvironment, which leads to the failure of immune systems such as T cells to kill tumor cells normally, thus achieving immune escape.

The mechanism of PD-1 or PD-L1 immunotherapy is to design specific monoclonal antibodies against PD-1 or PD-L1, prevent the recognition of PD-1 and PD-L1, and restore the normal function of T cells, so that T cells can effectively kill tumor cells. Currently, many therapeutic antibodies have been developed against this signaling pathway, such as Pembrolizumab and Nivolumab. However, during treating, there are widely existing phenomena such as low response rate and susceptible drug resistance. Therefore, an anti-tumor PD-1 antibody with stable structure, excellent therapeutic effect and suitable for large-scale industrial production is urgently needed.

### SUMMARY OF THE INVENTION

The present application provides an isolated antigen-binding protein having one or more of the following properties: (1) capable of binding to PD-1 protein derived from a primate with a *K_{D}* value of 1×10⁸ M or less; (2) capable of blocking the binding of PD-1 protein to PD-L1 protein; (3) capable of blocking the binding of PD-1 protein to PD-L2 protein; and (4) capable of stimulating the secretion of cytokines in immune cells, for example, stimulate lymphocytes to secrete IL-2.

In some embodiments, the primate comprises a human and/or a monkey.

In some embodiments, the isolated antigen-binding protein includes an antibody or antigen-binding fragment thereof.

In some embodiments, the antigen-binding fragment includes Fab, Fab', F(ab)₂, Fv fragment, F(ab')₂, scFv, di-scFv, VHH and/or dAb.

In some embodiments, the antibody is selected from the group consisting of a monoclonal antibody, chimeric antibody, humanized antibody and fully human antibody.

In some embodiments, the isolated antigen-binding protein is able to compete with a reference antibody for binding to PD-1 protein, wherein the reference antibody comprises a heavy chain variable region VH and a light chain variable region VL, the VH of the reference antibody comprises HCDR1, HCDR2 and HCDR3, the VL of the reference antibody comprises LCDR1, LCDR2 and LCDR3, and the reference antibody comprises any one of groups of amino acid sequences selected from: (1) HCDR1: SEQ ID NO: 1, HCDR2: SEQ ID NO: 2, HCDR3: SEQ ID NO: 3, LCDR1: SEQ ID NO: 8, LCDR2: SEQ ID NO: 9, and LCDR3: SEQ ID NO: 10; (2) HCDR1: SEQ ID NO: 27, HCDR2: SEQ ID NO: 28, HCDR3: SEQ ID NO: 29, LCDR1: SEQ ID NO: 34, LCDR2: SEQ ID NO: 35, and LCDR3: SEQ ID NO: 36; (3) HCDR1: SEQ ID NO: 1, HCDR2: SEQ ID NO: 85, HCDR3: SEQ ID NO: 3, LCDR1: SEQ ID NO: 8, LCDR2: SEQ ID NO: 9, and LCDR3: SEQ ID NO: 10.

In some embodiments, the isolated antigen-binding protein comprises HCDR3, and the HCDR3 comprises an amino acid sequence as set forth in SEQ ID NO: 3 or 29.

In some embodiments, the isolated antigen-binding protein comprises HCDR2, and the HCDR2 comprises an amino acid sequence as set forth in SEQ ID NO: 2, 28 or 85.

In some embodiments, the isolated antigen-binding protein comprises HCDR1, and the HCDR1 comprises an amino acid sequence as set forth in SEQ ID NO: 1 or 27.

In some embodiments, the isolated antigen-binding protein comprises HCDR1, HCDR2 and HCDR3, wherein the HCDR1 comprises an amino acid sequence as set forth in SEQ ID NO: 1 or SEQ ID NO: 27, the HCDR2 comprises an amino acid sequence as set forth in SEQ ID NO: 2, SEQ ID NO: 28 or SEQ ID NO: 85, and the HCDR3 comprises an amino acid sequence as set forth in SEQ ID NO: 3 or SEQ ID NO: 29.

In some embodiments, the isolated antigen-binding protein comprises HCDR1, HCDR2 and HCDR3, and the HCDR1, HCDR2 and HCDR3 are selected from any one of groups of amino acid sequences as follows: (1) HCDR1: SEQ ID NO: 1, HCDR2: SEQ ID NO: 2 and HCDR3: SEQ ID NO: 3; (2) HCDR1: SEQ ID NO: 27, HCDR2: SEQ ID NO: 28 and HCDR3: SEQ ID NO: 29; and (3) HCDR1: SEQ ID NO: 1, HCDR2: SEQ ID NO: 85 and HCDR3: SEQ ID NO: 3.

In some embodiments, the isolated antigen-binding protein comprises a heavy chain variable region VH, wherein the VH comprises a framework region H-FR1, and the C-terminus of the H-FR1 is directly or indirectly linked to the N-terminus of the HCDR1, and the H-FR1 comprises an amino acid sequence as set forth in SEQ ID NO: 57 or SEQ ID NO: 58.

In some embodiments, the H-FR1 comprises an amino acid sequence as set forth in any one of SEQ ID NO: 4, SEQ ID NO: 17, SEQ ID NO: 30 and SEQ ID NO: 43.

In some embodiments, the VH comprises a framework region H-FR2, the H-FR2 is located between the HCDR1 and the HCDR2, and the H-FR2 comprises an amino acid sequence as set forth in SEQ ID NO: 59 or SEQ ID NO: 60.

In some embodiments, the H-FR2 comprises an amino acid sequence as set forth in any one of SEQ ID NO: 5, SEQ ID NO: 18, SEQ ID NO: 31 and SEQ ID NO: 44.

In some embodiments, the VH comprises a framework region H-FR3, the H-FR3 is located between the HCDR2 and the HCDR3, and the H-FR3 comprises an amino acid sequence as set forth in SEQ ID NO: 61 or SEQ ID NO: 62.

In some embodiments, the H-FR3 comprises an amino acid sequence as set forth in any one of SEQ ID NO: 6, SEQ ID NO: 19, SEQ ID NO: 32 and SEQ ID NO: 45.

In some embodiments, the VH comprises a framework region H-FR4, the N-terminus of the H-FR4 is linked to the C-terminus of the HCDR3, and the H-FR4 comprises an amino acid sequence as set forth in SEQ ID NO: 63 or SEQ ID NO: 64.

In some embodiments, the H-FR4 comprises an amino acid sequence as set forth in any one of SEQ ID NO: 7, SEQ ID NO: 20, SEQ ID NO: 33 and SEQ ID NO: 46.

In some embodiments, the isolated antigen-binding protein comprises H-FR1, H-FR2, H-FR3 and H-FR4, wherein the H-FR1 comprises an amino acid sequence as set forth in any one of SEQ ID NO: 4, SEQ ID NO: 17, SEQ ID NO: 30 and SEQ ID NO: 43, the H-FR2 comprises an amino acid sequence as set forth in any one of SEQ ID NO: 5, SEQ ID NO: 18, SEQ ID NO: 31 and SEQ ID NO: 44, the H-FR3 comprises an amino acid sequence as set forth in any one of SEQ ID NO: 6, SEQ ID NO: 19, SEQ ID NO: 32 and SEQ ID NO: 45 and the H-FR4 comprises an amino acid sequence as set forth in any one of SEQ ID NO: 7, SEQ ID NO: 20, SEQ ID NO: 33 and SEQ ID NO: 46.

In some embodiments, the isolated antigen-binding protein comprises H-FR1, H-FR2, H-FR3 and H-FR4, and the H-FR1, H-FR2, H-FR3 and H-FR4 are selected from any one of groups of amino acid sequences as follows:
(1) H-FR1: SEQ ID NO: 4, H-FR2: SEQ ID NO: 5, H-FR3: SEQ ID NO: 6 and H-FR4: SEQ ID NO: 7;
(2) H-FR1: SEQ ID NO: 17, H-FR2: SEQ ID NO: 18, H-FR3: SEQ ID NO: 19 and H-FR4: SEQ ID NO: 20;
(3) H-FR1: SEQ ID NO: 30, H-FR2: SEQ ID NO: 31, H-FR3: SEQ ID NO: 32 and H-FR4: SEQ ID NO: 33; and
(4) H-FR1: SEQ ID NO: 43, H-FR2: SEQ ID NO: 44, H-FR3: SEQ ID NO: 45 and H-FR4: SEQ ID NO: 46.

In some embodiments, the isolated antigen-binding protein comprises a heavy chain variable region VH, and the VH comprises an amino acid sequence as set forth in SEQ ID NO: 65 or SEQ ID NO: 66.

In some embodiments, the VH comprises an amino acid sequence as set forth in any one of SEQ ID NO: 15, SEQ ID NO: 25, SEQ ID NO: 41, SEQ ID NO: 51 and SEQ ID NO: 86.

In some embodiments, the isolated antigen-binding protein comprises a heavy chain constant region, and the heavy chain constant region is derived from a human IgG constant region.

In some embodiments, the heavy chain constant region is derived from a human IgG4 constant region, and the human IgG4 constant region comprises an amino acid sequence as set forth in SEQ ID NO: 77 or SEQ ID NO: 81.

In some embodiments, the isolated antigen-binding protein comprises an antibody heavy chain HC, and the HC comprises an amino acid sequence as set forth in SEQ ID NO: 79, SEQ ID NO: 83 or SEQ ID NO: 87.

In some embodiments, the isolated antigen-binding protein comprises LCDR3, and the LCDR3 comprises an amino acid sequence as set forth in SEQ ID NO: 10 or SEQ ID NO: 36.

In some embodiments, the isolated antigen-binding protein comprises LCDR2, and the LCDR2 comprises an amino acid sequence as set forth in SEQ ID NO: 9 or SEQ ID NO: 35.

In some embodiments, the isolated antigen-binding protein comprises LCDR1, and the LCDR1 comprises an amino acid sequence as set forth in SEQ ID NO: 8 or SEQ ID NO: 34.

In some embodiments, the isolated antigen-binding protein comprises LCDR1, LCDR2 and LCDR3, wherein the LCDR1 comprises an amino acid sequence as set forth in any one of SEQ ID NO: 8 and SEQ ID NO: 34, the LCDR2 comprises an amino acid sequence as set forth in any one of SEQ ID NO: 9 and SEQ ID NO: 35, and the LCDR3 comprises an amino acid sequence as set forth in any one of SEQ ID NO: 10 and SEQ ID NO: 36.

In some embodiments, the isolated antigen-binding protein comprises LCDR1, LCDR2 and LCDR3, and the LCDR1, LCDR2 and LCDR3 are selected from any one of groups of amino acid sequences as follows: (1) LCDR1: SEQ ID NO: 34, LCDR2: SEQ ID NO: 35 and LCDR3: SEQ ID NO: 36; and (2) LCDR1: SEQ ID NO: 8, LCDR2: SEQ ID NO: 9 and LCDR3 : SEQ ID NO: 10.

In some embodiments, the isolated antigen-binding protein comprises a light chain variable region VL, wherein the VL comprises a framework region L-FR1, the C-terminus of the L-FR1 is directly or indirectly linked to the N-terminus of the LCDR1, and the L-FR1 comprises an amino acid sequence as set forth in SEQ ID NO: 69 or SEQ ID NO: 70.

In some embodiments, the L-FR1 comprises an amino acid sequence as set forth in any one of SEQ ID NO: 11, SEQ ID NO: 21, SEQ ID NO: 37 and SEQ ID NO: 47.

In some embodiments, the VL comprises a framework region L-FR2, the L-FR2 is located between the LCDR1 and the LCDR2, and the L-FR2 comprises an amino acid sequence as set forth in SEQ ID NO: 71 or SEQ ID NO: 72.

In some embodiments, the L-FR2 comprises an amino acid sequence as set forth in any one of SEQ ID NO: 12, SEQ ID NO: 22, SEQ ID NO: 38 and SEQ ID NO: 48.

In some embodiments, the VL comprises a framework region L-FR3, the L-FR3 is located between the LCDR2 and the LCDR3, and the L-FR3 comprises an amino acid sequence as set forth in SEQ ID NO: 73 or SEQ ID NO: 74.

In some embodiments, the L-FR3 comprises an amino acid sequence as set forth in any one of SEQ ID NO: 13, SEQ ID NO: 23, SEQ ID NO: 39 and SEQ ID NO: 49.

In some embodiments, the N-terminus of the L-FR4 is linked to the C-terminus of the LCDR3, and the L-FR4 comprises an amino acid sequence as set forth in SEQ ID NO: 75 or SEQ ID NO: 76.

In some embodiments, the L-FR4 comprises an amino acid sequence as set forth in any one of SEQ ID NO: 14, SEQ ID NO: 24, SEQ ID NO: 40 and SEQ ID NO: 50.

In some embodiments, the isolated antigen-binding protein comprises L-FR1, L-FR2, L-FR3 and L-FR4, wherein the L-FR1 comprises an amino acid sequence as set forth in any one of SEQ ID NO: 11, SEQ ID NO: 21, SEQ ID NO: 37 and SEQ ID NO: 47, the L-FR2 comprises an amino acid sequence as set forth in any one of SEQ ID NO: 12, SEQ ID NO: 22, SEQ ID NO: 38 and SEQ ID NO: 48, the L-FR3 comprises an amino acid sequence as set forth in any one of SEQ ID NO: 13, SEQ ID NO: 23, SEQ ID NO: 39 and SEQ ID NO: 49, and the L-FR4 comprises an amino acid sequence as set forth in any one of SEQ ID NO: 14, SEQ ID NO: 24, SEQ ID NO: 40 and SEQ ID NO: 50.

In some embodiments, the isolated antigen-binding protein comprises L-FR1, L-FR2, L-FR3 and L-FR4, and the L-FR1, L-FR2, L-FR3 and L-FR4 are selected from any one of groups of amino acid sequences as follows:
(1) L-FR1: SEQ ID NO: 11, L-FR2: SEQ ID NO: 12, L-FR3: SEQ ID NO: 13 and L-FR4: SEQ ID NO: 14;
(2) L-FR1: SEQ ID NO: 21, L-FR2: SEQ ID NO: 22, L-FR3: SEQ ID NO: 23 and L-FR4: SEQ ID NO: 24;
(3) L-FR1: SEQ ID NO: 37, L-FR2: SEQ ID NO: 38, L-FR3: SEQ ID NO: 39 and L-FR4: SEQ ID NO: 40; and
(4) L-FR1: SEQ ID NO: 47, L-FR2: SEQ ID NO: 48, L-FR3: SEQ ID NO: 49 and L-FR4: SEQ ID NO: 50.

In some embodiments, the isolated antigen-binding protein comprises a light chain variable region VL, and the VL comprises an amino acid sequence as set forth in SEQ ID NO: 67 or SEQ ID NO: 68.

In some embodiments, the VL comprises an amino acid sequence as set forth in any one of SEQ ID NO: 16, SEQ ID NO: 26, SEQ ID NO: 42 and SEQ ID NO: 52.

In some embodiments, the isolated antigen-binding protein comprises an antibody light chain constant region, and the antibody light chain constant region comprises an amino acid sequence as set forth in SEQ ID NO: 78 or 82.

In some embodiments, the isolated antigen-binding protein comprises an antibody light chain LC, and the LC comprises an amino acid sequence as set forth in SEQ ID NO: 80 or 84.

In another aspect, the present application provides a polypeptide comprising the isolated antigen-binding protein.

In another aspect, the present application provides an immunoconjugate comprising the isolated antigen-binding protein or the polypeptide.

In another aspect, the present application provides an isolated nucleic acid molecule(s) encoding the isolated antigen-binding protein.

In another aspect, the present application provides a vector comprising the nucleic acid molecule.

In another aspect, the present application provides a cell comprising the nucleic acid molecule or the vector.

In another aspect, the present application provides a method for preparing the isolated antigen-binding protein, comprising culturing the cell under conditions that the isolated antigen-binding protein can be expressed.

In another aspect, the present application provides a pharmaceutical composition comprising the isolated antigen-binding protein, the nucleic acid molecule, the vector and/or the cell, and optionally a pharmaceutically acceptable carrier.

In another aspect, the present application provides the use of the isolated antigen-binding protein, the polypeptide, the immunoconjugate, the nucleic acid molecule, the vector, the cell and/or the pharmaceutical composition in the manufacture of a medicament for preventing, alleviating and/or treating a tumor.

In another aspect, the present application provides a method for preventing, alleviating or treating a tumor, comprising administering the isolated antigen-binding protein, the polypeptide, the immunoconjugate, the nucleic acid molecule, the vector, the cell and/or the pharmaceutical composition to a subject in need.

In another aspect, the present application provides the isolated antigen-binding protein, for use in preventing, alleviating or treating a tumor.

In some embodiments, the tumor comprises a tumor with a high expression of PD-1 or PD-L1.

In some embodiments, the tumor comprises a solid tumor and/or a non-solid tumor.

In some embodiments, the tumor comprises melanoma, lung cancer, renal cancer, esophageal cancer, head and neck cancer, lymphoma, liver cancer and/or gastric cancer.

In another aspect, the present application provides a method of inhibiting the binding of PD-1 protein to PD-L1 protein, comprising administering the isolated antigen-binding protein, the polypeptide, the immunoconjugate, the nucleic acid molecule, the vector, the cell and/or the pharmaceutical composition.

In another aspect, the present application provides a method of inhibiting the binding of PD-1 protein to PD-L2 protein, comprising administering the isolated antigen-binding protein, the polypeptide, the immunoconjugate, the nucleic acid molecule, the vector, the cell and/or the pharmaceutical composition.

In another aspect, the present application provides a method for stimulating the secretion of cytokines in immune cells, comprising administering the isolated antigen-binding protein, the polypeptide, the immunoconjugate, the nucleic acid molecule, the vector, the cell and/or the pharmaceutical composition.

In another aspect, the present application provides a method for detecting the presence and/or content of PD-1 protein, comprising administering the isolated antigen-binding protein, the polypeptide, the immunoconjugate, the nucleic acid molecule, the vector, the cell and/or the pharmaceutical composition.

In another aspect, the present application provides a kit comprising the isolated antigen-binding protein, the polypeptide, the immunoconjugate, the nucleic acid molecule, the vector, the cell and/or the pharmaceutical composition.

Other aspects and advantages of the present application will be readily apparent to those skilled in the art from the following detailed description. In the following detailed description, only exemplary embodiments of the present application are shown and described. As will be recognized by those skilled in the art, the contents of the present application enable those skilled in the art to modify the disclosed specific embodiments without departing from the spirit and scope of the present application to which the present application relates. Accordingly, the descriptions in the drawings and specification of the present application are only exemplary but not restrictive.

### BRIEF DESCRIPTION OF DRAWINGS

The specific features of the present application to which the present application relates are shown in the appended claims. The features and advantages of the present application to which the present application relates will be better understood by referring to the exemplary embodiments and drawings described in detail below. A brief description of the attached drawings is as follows:
Fig. 1 shows that the antigen-binding proteins 1910h3hzL3H4 and 41D2HzL4H3 described in the present application bind to human PD-1 protein on the surface of CHOK1 cell.
Fig. 2 shows that the antigen-binding proteins 1910h3hzL3H4 and 41D2HzL4H3 described in the present application bind to cynomolgus monkey PD-1 protein on the surface of CHOK1 cell.
Fig. 3 shows that the antigen-binding proteins 1910h3hzL3H4 and 41D2HzL4H3 described in the present application block the binding of human PD-L1 to human PD-1 protein on the surface of CHOK1.
Fig. 4 shows that the antigen-binding proteins 1910h3hzL3H4 and 41D2HzL4H3 described in the present application block the binding of human PD-L2 to human PD-1 protein on the surface of CHOK1 cell.
Fig. 5 shows that the antigen-binding proteins 1910h3hzL3H4 and 41D2HzL4H3 described in the present application stimulate the secretion of cytokine IL-2 in mixed lymphocyte reaction.
Fig. 6 shows the binding results of candidate antibodies to human PD-1 on the cell surface.
Fig. 7 shows the binding results of the candidate antibodies to monkey PD-1 on the cell surface.
Fig. 8 shows the results of the candidate antibodies activating mixed lymphocytes to release IL-2.

### DETAILED DESCRIPTION OF THE INVENTION

The embodiments of the present application will be described in the following by specific examples, and those skilled in the art can easily understand other advantages and effects of the present application from the disclosure in this specification.

### Definition of Terms

In the present application, the term "PD-1" generally refers to the programmed death 1 receptor, which is also be called "programmed death 1", "CD279", "differentiation cluster 279", "PD1", "PDCD1" or "CD297". PD-1 protein usually comprises an extracellular IgV domain, a transmembrane domain and an intracellular tail. PD-1 is usually expressed on T cells, B cells, natural killer T cells, activated monocytes and dendritic cells (DC). PD-1 can bind to its ligands PD-L1 and PD-L2. The term "PD-1" encompasses any natural PD-1 or modified PD-1 derived from any vertebrates, including mammals, such as primates (for example, human or monkeys) and rodents (for example, mice or rats). The term encompasses "full-length", unprocessed PD-1 and any form of PD-1 produced by processing in cells. PD-1 can exist as a transmembrane protein or as a soluble protein. "PD-1" comprises complete PD-1 and fragments thereof, as well as functional variants, isoforms, homologues, derivatives and analogues of PD-1, and analogues with at least one common epitope with PD-1. PD-1 sequences are known in the art. For example, an exemplary full-length human PD-1 protein sequence can be found by NCBI Accession Number NP_005009.2, and an exemplary full-length cynomolgus monkey PD-1 protein sequence can be found by NCBI Accession Number NP_001271065 or Uniprot Accession Number B0LAJ3.

In the present application, the term "PD-L1" generally refers to the programmed death ligand 1 protein. PD-L1 is also called differentiation cluster 274 (CD274) or B7 homologue 1 (B7-H1), and it is a protein encoded by (human) CD274 gene. PD-L1 can bind to its receptor, such as programmed death 1 (PD-1). The complexation of PD-L1 and PD-1 plays an immunosuppressive role by inhibiting T cell proliferation and producing cytokines IL-2 and IFN-γ. The term "PD-L1" encompasses any natural PD-L1 or modified PD-1 derived from any vertebrates, including mammals, such as primates (for example, human or monkeys) and rodents (for example, mice or rats). The term encompasses "full-length", unprocessed PD-L1 and any form of PD-L1 produced by processing in cells. PD-L1 can exist as a transmembrane protein or as a soluble protein. The term also encompasses naturally occurring variants of PD-L1, such as splicing variants or allelic variants. The basic structure of PD-L1 comprises four domains: extracellular Ig-like V domain and Ig-like C2 domain, transmembrane domain and cytoplasmic domain. PD-L1 sequences are known in the art. For example, information about human PD-L1 gene (including genomic DNA sequence) can be found by NCBI Gene ID No.29126. The amino acid sequence of an exemplary full-length human PD-L1 protein can be found by NCBI Accession Number NP_054862 or UniProt Accession Number Q9NZQ7.

In the present application, the term "PD-L2" generally refers to the programmed death ligand 2 protein, which can also be called "programmed death 2", "CD273", "differentiation cluster 273", "B7-DC" or "PDCD1LG2". The term "PD-L2" encompasses any natural PD-L2 or modified PD-L2 derived from any vertebrate, including mammals, such as primates (for example, human or monkeys) and rodents (for example, mice or rats). The term encompasses "full-length", unprocessed PD-1 and any form of PD-L2 produced by processing in cells. PD-L2 can exist as a transmembrane protein or as a soluble protein. "PD-L2" comprises complete PD-L2 and fragments thereof, as well as functional variants, isoforms, homologues, derivatives, analogues of PD-L2, and analogues with at least one common epitope with PD-L2. PD-L2 sequences are known in the art. For example, an exemplary full-length human PD-L2 protein sequence can be found by NCBI Accession Number NP_079515.2.

In the present application, the term "antigen-binding protein" generally refers to a protein containing a portion that binds to an antigen, and optionally a scaffold or skeleton part portion that allows the portion that binds to an antigen with a conformation that promotes the binding of the antigen-binding protein to an antigen. An antigen-binding protein may typically comprise an antibody light chain variable region (VL), an antibody heavy chain variable region (VH) or both of them, and functional fragments thereof. The variable regions of heavy and light chains contain binding domains that interact with antigens. Examples of antigen-binding proteins include but not limited to antibodies, antigen-binding fragments, immunoconjugates, multispecific antibodies (e.g., bispecific antibodies), antibody fragments, antibody derivatives, antibody analogs or fusion proteins, or the like, as long as they show the required antigen-binding activity.

In the present application, the term "antibody" generally refers to an immunoglobulin that is reactive to a specified protein or peptide or fragment thereof. Antibodies can be antibodies from any class, including but not limited to IgG, IgA, IgM, IgD and IgE, and antibodies from any subclass (e.g. IgG1, IgG2, IgG3, and IgG4). The antibody may comprise a heavy chain constant region selected from IgG1, IgG2, IgG3, or IgG4, for example. The antibody may also comprise a light chain selected from kappa (κ) or lambda (λ), for example. The antibodies of the present application can be derived from any species.

In the present application, the term "antigen-binding fragment" generally refers to a portion of an antibody molecule, which contains an amino acid residue that interacts with an antigen and gives the antibody specificity and affinity to an antigen. Examples of antigen-binding fragments may include but not limited to Fab, Fab', F(ab)2, a Fv fragment, F(ab')₂, scFv, di-scFv and/or dAb. In the present application, the term "Fab" generally refers to a fragment containing a heavy chain variable domain and a light chain variable domain, and also contains a constant domain of the light chain and a first constant domain (CH1) of the heavy chain; the term "Fab'" generally refers to a fragment different from Fab by adding a small amount of residues (including one or more cysteine from the antibody hinge region) to the carboxyl end of the CH1 domain of the heavy chain; the term "F(ab')₂" generally refers to the dimer of Fab', which contains antibody fragments of two Fab fragments connected by a disulfide bridge on the hinge region. The term "Fv" generally refers to a smallest antibody fragment containing complete antigen recognition and binding sites. In some embodiments, the fragment can be composed of a dimer with a heavy chain variable region and a light chain variable region in closely non-covalent bonding; the term "dsFv" generally refers to disulfide-stabilized Fv fragments, in which the bond between a single light chain variable region and a single heavy chain variable region is disulfide bond. The term "dAb fragment" generally refers to an antibody fragment composed of a VH domain. In the present application, the term "scFv" generally refers to a monovalent molecule formed by covalent connection and pairing of a heavy chain variable domain and a light chain variable domain of an antibody through a flexible peptide linker; such scFv molecule can comprise the general structure: NH₂-VL- linker-VH-COOH or NH₂-VH-linker-VL-COOH.

In the present application, the term "variable region" or "variable domain" generally refers to the domain of antibody heavy chain or light chain involved in the binding of antibody to antigen. In the present application, the term "variable" generally means that some portions of the sequence of the variable domain of an antibody change strongly, forming the binding and specificity of various specific antibodies to their specific antigens. Variability is distributed in the whole variable region of antibody unevenly. It mainly exists in three segments in the light chain variable region and the heavy chain variable region, which are called complementarity determining region (CDR) or high variable region (HVR), namely LCDR1, LCDR2, LCDR3, HCDR1, HCDR2 and HCDR3 respectively. The more highly conservative portion of the variable domain is called a framework region (FR). The variable domains of natural heavy chain and light chain comprise four FR regions (H-FR1, H-FR2, H-FR3, H-FR4, L-FR1, L-FR2, L-FR3, L-FR4) respectively, and most of them have β-folding configuration and are connected by three CDR structural loop regions. CDRs in each chain are close together through FR regions, and together with the CDRs from the other chain form an antigen binding site of the antibody.

In this field, variable regions of antibodies or CDRs of antibodies can be numbered by various methods, such as Kabat numbering scheme and definition rules based on sequence variability (see Kabat et al., protein sequence in immunology, 5th Edition, National Institutes of Health, Bethesda, Maryland (1991)), Chothia numbering scheme and definition rules based on the position of structural loop regions (see, A1- Lazikani et al., J Mol Biol 273 :927-48,1997), IMGT numbering scheme and definition rules based on amino acid sequence alignment of germline V gene by efranc *et al.,* and Honneger's numbering scheme (AHo's), Martin numbering scheme, Gelfand numbering scheme, etc. See Mathieu Dondelinger et al., Understanding the Significance and Implications of Antibody Numbering and Antigen-Binding Surface/Residue Definition, Front. Immunol., 16 October 2018.

In the present application, the term "monoclonal antibody" generally refers to an antibody obtained from a group of basically homogeneous antibodies, that is, each antibody constituting the group is the same, except for possible naturally occurring mutations and/or post-translational modifications (e.g., isomerization, amidation) that may exist in a very small amount. Monoclonal antibodies are highly specific and for a single antigenic site.

In the present application, the term "chimeric antibody" generally refers to an antibody in which the variable region is derived from one species and the constant region is derived from another species. Generally, the variable region is derived from an antibody of an experimental animal such as a rodent ("parent antibody"), and the constant region is derived from a human antibody, so that the obtained chimeric antibody is less likely to cause an adverse immune response in a human individual, compared with a parent (e.g., mouse-derived) antibody.

In the present application, the term "humanized antibody" generally refers to an antibody in which some or all of the amino acids other than the CDR region of a non-human antibody (e.g. a mouse antibody) are replaced by corresponding amino acids derived from human immunoglobulin. Addition, deletion, insertion, substitution or modification of amino acids in the CDR region can also be allowed, as long as they still retain the ability of antibodies to bind to specific antigens. A humanized antibody may optionally comprise at least a portion of the constant region of human immunoglobulin. A humanized antibody retains the antigen specificity similar to that of the original antibody. A "humanized" form of a non-human (e.g., murine) antibody may comprise a chimeric antibody derived from a sequence of a non-human immunoglobulin at a minimum. In some cases, the residues of CDR region in human immunoglobulin (receptor antibody) can be replaced by the residues of CDR region of a non-human species (donor antibody) (such as mice, rats, rabbits or non-human primates) with desired properties, affinity and/or ability. In some cases, the residues in the FR region of human immunoglobulin can be replaced by corresponding non-human residues. In addition, the humanized antibody may comprise amino acid modifications that are not present in the recipient antibody or in the donor antibody.

In the present application, the term "fully human antibody" generally refers to an antibody in which all portions (including the variable region and the constant region of the antibody) are encoded by genes of human origin. The methods to obtain fully human antibodies in this field may comprise phage display technology, transgenic mouse technology, ribosome display technology and RNA- polypeptide technology.

In the present application, the terms "binding", "specifically binding" or "specific for" generally refer to measurable and reproducible interactions, such as the binding of antigens with antibodies, which can determine the presence of a target in the presence of heterogeneous populations of molecules, including biological molecules. For example, an antibody binds to an epitope through its antigen-binding domain, and this binding requires some complementarity between the antigen-binding domain and the epitope. For example, an antibody that specifically binds to a target (which may be an epitope) is an antibody that binds to this target with greater affinity, binding affinity, and easier and/or longer duration than binding of the antibody to other targets. An antibody is called to "specifically bind" to an antigen when it is more likely to bind to an epitope through its antigen-binding domain than binding of the antibody to a random, unrelated epitope.

In the present application, the terms "KD" and *"K_{D}"* can be used interchangeably and usually refer to the equilibrium dissociation constant, and "KD" is a ratio of the dissociation rate constant (k_{dis}, also known as "off-rate (k_{off})" or "kd") to the association rate constant (kₒₙ, also known as "bonding rate (kₒₙ)" or "ka"). Association rate constant (kₒₙ), dissociation rate constant (k_{dis}) and equilibrium dissociation constant (KD) can be used to express the binding affinity of antigen-binding proteins (e.g. antibodies) to antigens. Methods for determining the association and dissociation rate constants are well known in the art, including but not limited to Biolayer interferometry (BLI), Radioimmunoassay (RIA), Equilibrium Dialysis, Surface Plasmon Resonance (SPR), Fluorescence Resonance Energy Transfer (FRET), co-immunoprecipitation (Co-IP) and protein Chip Technology. If measured under different conditions (e.g. salt concentration and pH), the measured affinity of a specific protein-protein interaction may be different.

In the present application, the term "primate" usually refers to monkey and ape species, and includes monkey species, such as the monkeys from the *Macaca* (such as, cynomolgus (*Macaca fascicularis*) and/or rhesus monkey (*Macaca mulatta*)) and baboon (*Papio ursinus*), as well as marmoset (a species from *Callithrix*), squirrel monkey (a species from Saimiri), and tamarin (a species from *Saguinus*), and ape species, such as chimpanzee (*Pan troglodytes*), and also includes *Homo sapiens.*

In the present application, the terms "polypeptide" or "protein" are used interchangeably and generally refer to polymers of amino acid residues. The term also applies to amino acid polymers in which one or more amino acid residues are analogs or mimetics of corresponding naturally occurring amino acids, as well as naturally occurring amino acid polymers. The term may also comprise modified amino acid polymers, for example, by adding sugar residues to form glycoproteins or modification of phosphorylation. Polypeptides and proteins can be produced by naturally occurring and non-recombinant cells or genetically engineered or recombinant cells, and may comprise molecules with amino acid sequences of natural protein, or molecules with deletion, addition and/or substitution of one or more amino acids of natural sequences. The terms "polypeptide" and "protein" particularly comprise deletion, addition and/or substitution of one or more amino acids of a sequence of the antigen-binding protein described in the present application.

In the present application, the term "isolated" generally refers to biological materials (e.g. viruses, nucleic acids, or protein) that are substantially free of components normally associated with or interacting with their naturally occurring environment. The isolated biological materials optionally includes additional materials that are not found in its natural environment (e.g., nucleic acids or proteins). In the present application, when it comes to protein, "isolation" usually means that the molecule is isolated and separated from the whole organism in which the molecule is found to exist naturally, or there are basically no other biomacromolecules of the same type. When a nucleic acid molecule is referred to, it is completely or partially separated from the sequence that naturally binds to it, or the nucleic acid has a heterologous sequence that binds to it, or the nucleic acid is separated from the chromosome.

In the present application, the term "immunoconjugate" generally refers to the substance formed by the connection of antigen-binding protein with other active agents, which may be a small molecular active agent, such as a chemotherapeutic agent, a toxin, an immunotherapy agent, an imaging probe or a fluorescent probe.

In the present application, the term "nucleic acid" molecule generally refers to any length of isolated form of nucleotide, deoxynucleotide or ribonucleotide or its analogues isolated from its natural environment or artificially synthesized.

In the present application, the term "vector" generally refers to a nucleic acid molecule capable of self-replication in a suitable host, which transfers the inserted nucleic acid molecule into and/or between host cells. The vector may comprise a vector mainly used for inserting DNA or RNA into cells, a vector mainly used for replicating DNA or RNA, and a vector mainly used for transcription and/or expression of translation of DNA or RNA. The vector also comprises a vector with a plurality of the above functions. The vector can be a polynucleotide that can be transcribed and translated into a polypeptide when introduced into a suitable host cell. Generally, the vector can produce a desired expression product by culturing a suitable host cell comprising the vector.

In the present application, the term "cell" generally refers to an individual cell, cell line or cell culture that may comprise or already has comprised a plasmid or vector including a nucleic acid molecule described in the present application, or that can express an antigen-binding protein described in the present application. The cells may comprise progeny of a single host cell. Because of natural, unexpected or intentional mutation, the progeny cells may not be exactly the same as the original parent cells in morphology or genome, but they can express the antibody or antigen-binding fragment thereof described in the present application. The cells can be obtained by transfecting cells in vitro using the vector described in the present application. The cells can be prokaryotic cells (e.g. *Escherichia coli*) or eukaryotic cells (e.g. yeast cells, e.g. COS cells, China hamster ovary (CHO) cells, HeLa cells, HEK293 cells, COS-1 cells, NS0 cells or myeloma cells). In some cases, the cells may be mammalian cells. For example, the mammalian cells may be CHO-K1 cells.

In the present application, the term "pharmaceutical composition" generally refers to a preparation that exists in a form that allows the biological activity of an active ingredient to be effective, and does not contain additional ingredients with unacceptable toxicity to a subject to whom the composition is to be administered.

In the present application, the term "treatment" generally refers to a desired change in the natural course of the individual being treated, and may be clinical intervention to achieve prevention and treatment or in the process of clinical lesions. Desirable therapeutic effects include but not limited to preventing the occurrence or recurrence of the disease, relieving symptoms, weakening any direct or indirect pathological consequences of the disease, preventing metastasis, reducing the rate of disease progression, improving or alleviating the disease condition and alleviating or improving the prognosis. In some cases, antibodies (e.g., anti-PD-1 antibodies) can be used to delay disease development or slow disease progression.

In the present application, the term "administration" generally refers to a method of administering a certain dose of a compound (e.g., an anticancer therapeutic agent) or a pharmaceutical composition (e.g., a pharmaceutical composition comprising an anticancer therapeutic agent) to a subject (e.g., a patient). Administration can be carried out by any suitable means, including parenteral, intrapulmonary and intranasal administration, and (if necessary for local treatment) intralesional administration. Parenteral infusion comprises, for example, intramuscular, intravenous, intra-arterial, intraperitoneal or subcutaneous administration.

In the present application, the term "tumor" generally refers to the growth and proliferation of all neoplastic cell (either malignant or benign) and all precancerous and cancerous cells and tissues. In the present application, the tumor can be a tumor with a high expression of PD-1 or PD-L1 in cells and tissues. Tumors may comprise solid tumors and/or non-solid tumors (e.g., hematomas, lymphomas).

In the present application, the term "homologue" generally refers to an amino acid sequence or nucleotide sequence that has certain homology with the wild-type amino acid sequence and the wild-type nucleotide sequence. The term "homology" can be equivalent to sequence "identity". Homologous sequences may comprise amino acid sequences that may be at least 80%, 85%, 90%, 99.1%, 99.2%, 99.3%, 99.4%, 99.5%, 99.6%, 99.7%, 99.8% or 99.9% identical to the subject sequence. Typically, the homologue will comprise the same active site or the like as the subject amino acid sequence. Homology may be considered in terms of similarity (i.e., amino acid residues having similar chemical properties/functions), or may be expressed in terms of sequence identity. In the present application, a sequence having percentage identity in any one of the mentioned SEQ ID NO of a mentioned amino acid sequence or nucleotide sequence refers to a sequence having the percentage identity over the entire length of the mentioned SEQ ID NO.

In the present application, the term "between" generally means that the C-terminus of an amino acid fragment is directly or indirectly linked to the N-terminus of a first amino acid fragment, and its N-terminus is directly or indirectly linked to the C-terminus of a second amino acid fragment. In the light chain, for example, the N-terminus of the L-FR2 is directly or indirectly linked to the C-terminus of the LCDR1, and the C-terminus of the L-FR2 is directly or indirectly linked to the N-terminus of the LCDR2. For another example, the N-terminus of the L-FR3 is directly or indirectly linked to the C-terminus of the LCDR2, and the C-terminus of the L-FR3 is directly or indirectly linked to the N-terminus of the LCDR3. In the heavy chain, for example, the N-terminus of the H-FR2 is directly or indirectly linked to the C-terminus of the HCDR1, and the C-terminus of the H-FR2 is directly or indirectly linked to the N-terminus of the HCDR2. For another example, the N-terminus of the H-FR3 is directly or indirectly linked to the C-terminus of the HCDR2, and the C-terminus of the H-FR3 is directly or indirectly linked to the N-terminus of the HCDR3. In the present application, the "first amino acid fragment" and the "second amino acid fragment" can be any one of the same or different amino acid fragments.

In the present application, the term "comprising" generally refers to the meaning of including, encompassing, containing or covering. In some cases, it also means "being" and "being consist of".

In the present application, the term "about" generally refers to a change in the range of 0.5%-10% above or below a specified value, such as a change in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### Detailed Description of the Present Application

### Antigen-Binding Protein

In an aspect, the present application provides an isolated antigen-binding protein, and in the present application, the isolated antigen-binding protein can bind to PD-1 derived from a primate with a KD value of 1 × 10⁻⁸ M or less. The binding affinity of primate PD-1 antigen-binding protein to PD-1 can be determined by any method known in the art. In some cases, the binding affinity can be determined by surface plasmon resonance (SPR), enzyme-linked immunosorbent assay (ELISA), binding antigen precipitation method, equilibrium dialysis method and Biolayer interferometry (BLI). In some cases, the binding affinity and KD value of PD-1 antigen-binding protein to PD-1 can be determined by Biolayer interference (BLI). For example, an ForteBio Octet molecular interaction analyzer can be used to analyze the binding kinetics between antigens and antibodies.

In the present application, the isolated antigen-binding protein can bind to PD-1 derived from a primate with a KD value of 1 × 10⁻⁸ M or less. For example, PD-L1 derived from humans may be bound to with the KD value of a value of about 1 × 10 ⁻⁸ M or less, about 9 × 10⁻⁹ M or less, about 8 × 10⁻⁹ M or less, about 7 × 10⁻⁹ M or less, about 6 × 10⁻⁹ M or less, about 5 × 10⁻⁹ M or less, about 4 × 10⁻⁹ M or less, about 3 × 10⁻⁹ M or less, about 2 × 10⁻⁹ M or less, and about 1 × 10⁻⁹ M or less, for example, measured by using a FortieBio Octet molecular interaction analyzer.

In another case, the binding activity of PD-1 antigen-binding protein described in the present application to PD-1 can be measured by flow cytometry or enzyme-linked immunosorbent assay.

For example, in a FACS test, host cells stably expressing human PD-1 (such as CHOK1 cells) are used, and the EC50 value of the PD-1 antigen-binding protein binding to PD-1 is between about 0.0001nM and about 100nM, for example, between about 0.001nM and about 10nM, between about 0.001nM and about 5nM, between about 0.001nM and about 1nM, between about 0.01nM and about 1nM, between about 0.01nM and about 1.2nM, or between about O.OlnM and about 1.5nM.

For example, in a FACS test, host cells stably expressing mokey PD-L1 (such as CHOK1 cells) are used, and the EC50 value of PD-L1 antigen-binding protein binding to PD-L1 is between about 0.0001nM and about 100nM, for example, between about 0.001nM and about 10nM, between about 0.001nM and about 5nM, between about 0.01nM and about 1nM, between about 0.02nM and about 1.0nM, and between about 0.1nM and about 1.0nM.

In another aspect, the antigen-binding protein described in the present application can block the binding of PD-1 to PD-L1. In some cases, the blocking of the binding of PD-1 to PD-L1 by the antigen-binding protein may be determined by flow cytometry FACS or euzymelinked immunosorbent assay (ELISA).

For example, host cells stably expressing human PD-1 (such as HEK293 cells) are first incubated with decreasing amounts of unlabeled antigen-binding protein, and then incubated with biotin-labeled PD-L1 protein. Then, the cells are analyzed using FACS to confirm that the antigen-binding protein blocks the binding of PD-1 to PD-L1. For example, the IC50 value is between about 0.001nM and about 10nM, between about 0.001nM and about 5nM, between about 0.01nM and about 5nM, between about 0.1nM and about 1.0nM, and between about 0.5nM and about 1.5nM.

For example, host cells stably expressing human PD-1 (such as CHOK1 cells) are first incubated with decreasing amounts of unlabeled antigen-binding protein, and then incubated with biotin-labeled PD-L2 protein. Then, the cells are analyzed using FACS to confirm that the antigen-binding protein blocks the binding of PD-1 to PD-L2. For example, between about 0.001nM to about 10nM, about 0.001nM to about 5nM, about 0.1nM to about 2.5nM, and about 0.1nM to about 3nM.

In the present application, the isolated antigen-binding protein may comprise HCDR3, and the HCDR3 may comprise CDR3 of VH with an amino acid sequence as set forth in SEQ ID NO: 65.

In the present application, the isolated antigen-binding protein may comprise HCDR3, and the HCDR3 may comprise CDR3 of VH with an amino acid sequence as set forth in SEQ ID NO: 15 or 25. For example, the isolated antigen-binding protein may comprise HCDR3, and the HCDR3 may comprise an amino acid sequence as set forth in SEQ ID NO: 3.

In the present application, the isolated antigen-binding protein may comprise HCDR2, and the HCDR2 may comprise CDR2 of VH with an amino acid sequence as set forth in SEQ ID NO: 65.

In the present application, the isolated antigen-binding protein may comprise HCDR2, and the HCDR2 may comprise CDR2 of VH with an amino acid sequence as set forth in SEQ ID NO: 15 or 25. For example, the isolated antigen-binding protein may comprise HCDR2, and the HCDR2 may comprise an amino acid sequence as set forth in SEQ ID NO: 2.

In the present application, the isolated antigen-binding protein may comprise HCDR2, and the HCDR2 may comprise CDR2 of VH with an amino acid sequence as set forth in SEQ ID NO: 86. For example, the isolated antigen-binding protein may comprise HCDR2, and the HCDR2 may comprise an amino acid sequence as set forth in SEQ ID NO: 85.

In the present application, the isolated antigen-binding protein may comprise HCDR1, and the HCDR1 may comprise CDR1 of VH with an amino acid sequence as set forth in SEQ ID NO: 65.

In the present application, the isolated antigen-binding protein may comprise HCDR1, and the HCDR1 may comprise CDR1 of VH with an amino acid sequence as set forth in SEQ ID NO: 15 or 25. For example, the isolated antigen-binding protein may comprise HCDR1, the HCDR1 may comprise an amino acid sequence as set forth in SEQ ID NO: 1.

In the present application, the isolated antigen-binding protein may comprise HCDR1, HCDR2 and HCDR3, the HCDR1 may comprise CDR1 of VH with an amino acid sequence as set forth in SEQ ID NO: 65, the HCDR2 may comprise CDR2 of VH with an amino acid sequence as set forth in SEQ ID NO: 65, and the HCDR3 may comprise CDR3 of VH with an amino acid sequence as set forth in SEQ ID NO: 65.

In the present application, the isolated antigen-binding protein may comprise HCDR1, HCDR2 and HCDR3, the HCDR1 may comprise CDR1 of VH with an amino acid sequence as set forth in SEQ ID NO: 15 or 25, the HCDR2 may comprise CDR2 of VH with an amino acid sequence as set forth in SEQ ID NO: 15 or 25, and the HCDR3 may comprise CDR3 of VH with an amino acid sequence as set forth in SEQ ID NO: 15 or 25.

In the present application, the isolated antigen-binding protein may comprise HCDR1, HCDR2 and HCDR3, the HCDR1 may comprise CDR1 of VH with an amino acid sequence as set forth in SEQ ID NO: 86, the HCDR2 may comprise CDR2 of VH with an amino acid sequence as set forth in SEQ ID NO: 86, and the HCDR3 may comprise CDR3 of VH with an amino acid sequence as set forth in SEQ ID NO: 86.

For example, the isolated antigen-binding protein may comprise CDR1 of VH with an amino acid sequence as set forth in SEQ ID NO: 15, may comprise CDR2 of VH with an amino acid sequence as set forth in SEQ ID NO: 15, and may comprise CDR3 of VH with an amino acid sequence as set forth in SEQ ID NO: 15.

For example, the isolated antigen-binding protein may comprise CDR1 of VH with an amino acid sequence as set forth in SEQ ID NO: 25, may comprise CDR2 of VH with an amino acid sequence as set forth in SEQ ID NO: 25, and may comprise CDR3 of VH with an amino acid sequence as set forth in SEQ ID NO: 25.

For example, the isolated antigen-binding protein described in the present application may comprise HCDR1, HCDR2 and HCDR3, the HCDR1 may comprise an amino acid sequence as set forth in SEQ ID NO: 1, the HCDR2 may comprise an amino acid sequence as set forth in SEQ ID NO: 2 and the HCDR3 may comprise an amino acid sequence as set forth in SEQ ID NO: 3.

For example, the isolated antigen-binding protein described in the present application may comprise HCDR1, HCDR2 and HCDR3, the HCDR1 may comprise an amino acid sequence as set forth in SEQ ID NO: 1, the HCDR2 may comprise an amino acid sequence as set forth in SEQ ID NO: 85 and the HCDR3 may comprise an amino acid sequence as set forth in SEQ ID NO: 3.

In the present application, the isolated antigen-binding protein may comprise H-FR1, and the H-FR1 may comprise an amino acid sequence as set forth in SEQ ID NO: 57: EVX₃LVESGGGLVKPGGSLX₁₉LX₂₁CAAS (SEQ ID NO: 57), where X₃ is K or Q, X₁₉ is K or R, and X₂₁ is A or S. For example, it may be divided according to Chothia rules.

In some cases, compared with an amino acid sequence as set forth in SEQ ID NO: 4, the H-FR1 may comprise at least an amino acid substitution at a position selected from the group consisting of amino acid substitutions at X₃, X₁₉ and/or X₂₁. For example, the H-FR1 may comprise an amino acid sequence as set forth in SEQ ID NO: 4 or 17.

In the present application, the isolated antigen-binding protein may comprise H-FR2, and the H-FR2 may comprise an amino acid sequence as set forth in SEQ ID NO: 59: DMSWVRQX₈PX₁₀KX₁₂LEWVX₁₇TI (SEQ ID NO: 59), where X₈ is A or T, X₁₀ is E or G, X₁₂ is G or R, and X₁₇ is A or S. For example, it may be divided according to Chothia rules.

In some cases, compared with an amino acid sequence as set forth in SEQ ID NO: 5, the H-FR2 may comprise at least an amino acid substitution at a position selected from the group consisting of amino acid substitutions at X₈, X₁₀, X₁₂ and/or X₁₇. For example, the H-FR2 may comprise an amino acid sequence as set forth in SEQ ID NO:5 or 18.

In the present application, the isolated antigen-binding protein may comprise H-FR3, and the H-FR3 may comprise an amino acid sequence as set forth in SEQ ID NO: 61: TYYX₄DSVKGRFTISRDNAKNX₂₁LYLQMX₂₇SLRX₃₁EDTAX₃₆YYCVS (SEQ ID NO: 61), where X₄ is A or P, X₂₁ is S or N, X₂₇ is N or S, X₃₁ is A or S, and X₃₆ is L or V For example, it may be divided according to Chothia rules.

In some cases, compared with an amino acid sequence as set forth in SEQ ID NO: 6, the H-FR3 may comprise at least an amino acid substitution at a position selected from the group consisting of amino acid substitutions at X₄, X₂₁, X₂₇, X₃₁ and/or X₃₆.

For example, the H-FR3 may comprise an amino acid sequence as set forth in SEQ ID NO: 6 or 19.

In the present application, the isolated antigen-binding protein may comprise H-FR4, and the H-FR4 may comprise an amino acid sequence as set forth in SEQ ID NO: 63: WGQGTX₆VTVSS (SEQ ID NO: 63), where X₆ is L or S. For example, it may be divided according to Chothia rules.

In some cases, compared with an amino acid sequence as set forth in SEQ ID NO: 7, and the H-FR3 may comprise at least an amino acid substitution at X₆. For example, the H-FR4 may comprise an amino acid sequence as set forth in SEQ ID NO: 7 or 20.

In the present application, the isolated antigen-binding protein may comprise a heavy chain variable region VH, and the VH may comprise an amino acid sequence as set forth in SEQ ID NO: 65: EVX₃LVESGGGLVKPGGSLX₁₉LX₂₁CAASGFTFSNYDMSWVRQX₄₀PX₄₂KX₄₄LEWVX₄₉ TISGGGSYTYYX₆₁DSVKGRFTISRDNAKNX₇₈LYLQMX₈₄SLRX₈₈EDTAX₉₃YYCVSPYY GMEYWGQGTX₁₁₁VTVSS (SEQ ID NO: 65), where X₃ is K or Q, X₁₉ is K or R, X₂₁ is A or S, X₄₀ is A or T, X₄₂ is E or G, X₄₄ is G or R, X₄₉ is A or S, X₆₁ is A or P, X₇₈ is N or S, X₈₄ is S or N, X₈₈ is A or S, X₉₃ is L or V, and X₁₁₁ is L or S. For example, it may be divided according to Chothia rules.

In some cases, compared with an amino acid sequence as set forth in SEQ ID NO: 15, the VH may comprise at least an amino acid substitution at a position selected from the group consisting of amino acid substitutions at X₃, X₁₉, X₂₁, X₄₀, X₄₂, X₄₄, X₄₉, X₆₁, X₇₈, X₈₄, X₈₈, X₉₃ and/or X₁₁₁.

For example, the VH may comprise an amino acid sequence as set forth in SEQ ID NO: 15 or 25. For example, the VH may comprise an amino acid sequence as set forth in SEQ ID NO: 86.

The isolated antigen-binding protein of the present application may comprise LCDR3, the LCDR3 may comprise CDR3 of VL with an amino acid sequence as set forth in SEQ ID NO: 67.

In the present application, the isolated antigen-binding protein may comprise LCDR3, and the LCDR3 may comprise CDR3 of VL with an amino acid sequence as set forth in SEQ ID NO: 16 or 26. For example, the isolated antigen-binding protein may comprise LCDR3, and the LCDR3 may comprise an amino acid sequence as set forth in SEQ ID NO: 10.

In the present application, the isolated antigen-binding protein may comprise LCDR2, and the LCDR2 may comprise CDR2 of VL with an amino acid sequence as set forth in SEQ ID NO: 67.

In the present application, the isolated antigen-binding protein may comprise LCDR2, and the LCDR2 may comprise CDR2 of VL with an amino acid sequence as set forth in SEQ ID NO: 16 or 26. For example, the isolated antigen-binding protein may comprise LCDR2, and the LCDR2 may comprise an amino acid sequence as set forth in SEQ ID NO: 9.

In the present application, the isolated antigen-binding protein may comprise LCDR1, and the LCDR1 may comprise CDR1 of VL with an amino acid sequence as set forth in SEQ ID NO: 67.

In the present application, the isolated antigen-binding protein may comprise LCDR1, and the LCDR1 may comprise CDR1 of VL with an amino acid sequence as set forth in SEQ ID NO: 16 or 26. For example, the isolated antigen-binding protein may comprise LCDR1, and the LCDR1 may comprise an amino acid sequence as set forth in SEQ ID NO: 8.

In the present application, the isolated antigen-binding protein may comprise LCDR1, LCDR2 and LCDR3, the LCDR1 may comprise CDR1 of VL with an amino acid sequence as set forth in SEQ ID NO: 67, the LCDR2 may comprise CDR2 of VL with an amino acid sequence as set forth in SEQ ID NO: 67, and the LCDR3 may comprise CDR3 of VL with an amino acid sequence as set forth in SEQ ID NO: 67.

In the present application, the isolated antigen-binding protein may comprise LCDR1, LCDR2 and LCDR3, the LCDR1 may comprise CDR1 of VL with an amino acid sequence as set forth in SEQ ID NO: 16 or 26, the LCDR2 may comprise CDR2 of VL with an amino acid sequence as set forth in SEQ ID NO: 16 or 26, and the LCDR3 may comprise CDR3 of VL with an amino acid sequence as set forth in SEQ ID NO: 16 or 26.

For example, the isolated antigen-binding protein may comprise CDR1 of VL with an amino acid sequence as set forth in SEQ ID NO: 16, may comprise CDR2 of VL with an amino acid sequence as set forth in SEQ ID NO: 16, and may comprise CDR3 of VL with an amino acid sequence as set forth in SEQ ID NO: 16.

For example, the isolated antigen-binding protein may comprise CDR1 of VL with an amino acid sequence as set forth in SEQ ID NO: 26, may comprise CDR2 of VL with an amino acid sequence as set forth in SEQ ID NO: 26, and may comprise CDR3 of VL with an amino acid sequence as set forth in SEQ ID NO: 26.

For example, the isolated antigen-binding protein described in the present application may comprise LCDR1, LCDR2 and LCDR3, the LCDR1 may comprise an amino acid sequence as set forth in SEQ ID NO: 8, the LCDR2 may comprise an amino acid sequence as set forth in SEQ ID NO: 9, and the LCDR3 may comprise an amino acid sequence as set forth in SEQ ID NO: 10.

In the present application, the isolated antigen-binding protein may comprise L-FR1, and the L-FR1 may comprise an amino acid sequence as set forth in SEQ ID NO: 69: DIX₃X₄TQSX₈X₉FX₁₁SX₁₃SVGDRVX₂₀TC (SEQ ID NO: 69), where X₃ is Q or V, X₄ is L or M, X₈ is P or H, X₉ is K or S, X₁₁ is L or M, X₁₃ is A or T, and X₂₀ is S or T. For example, it may be divided according to Chothia rules.

In some cases, compared with an amino acid sequence as set forth in SEQ ID NO: 11, the L-FR1 may comprise at least an amino acid substitution at a position selected from the group consisting of amino acid substitutions at X₃, X₄, X₈, X₉, X₁₁, X₁₃ and/or X₂₀.

For example, the L-FR1 may comprise an amino acid sequence as set forth in SEQ ID NO: 11 or 21.

In the present application, the isolated antigen-binding protein may comprise L-FR2, and the L-FR2 may comprise an amino acid sequence as set forth in SEQ ID NO: 71: WYQQKPGX₈X₉PKLLIY(SEQ ID NO: 71), where X₈ is K or Q, and X₉ is A or S. For example, it may be divided according to Chothia rules.

In some cases, compared with an amino acid sequence as set forth in SEQ ID NO: 12, the L-FR2 may comprise at least an amino acid substitution at a position selected from the group consisting of amino acid substitutions at X₈ and/or X₉.

For example, the L-FR2 may comprise an amino acid sequence as set forth in SEQ ID NO: 12 or 22.

In the present application, the isolated antigen-binding protein may comprise L-FR3, and the L-FR3 may comprise an amino acid sequence as set forth in SEQ ID NO: 73: GVPX₄RFX₇GSGSGTX₁₄FTLTISX₂₁X₂₂QX₂₄EDX₂₇AX₂₉YFC (SEQ ID NO: 73), where X₄ is D or S, X₇ is S or T, X₁₄ is D or E, X₂₁ is N or S, X₂₂ is L or V, X₂₄ is P or S, X₂₇ is F or L, and X₂₉ is D or T. For example, it may be divided according to Chothia rules.

In some cases, compared with an amino acid sequence as set forth in SEQ ID NO: 13, the L-FR3 may comprise at least an amino acid substitution at a position selected from the group consisting of amino acid substitutions at X₄, X₇, X₁₄, X₂₁, X₂₂, X₂₄, X₂₇ and/or X₂₉.

For example, the L-FR3 may comprise an amino acid sequence as set forth in SEQ ID NO: 13 or 23.

In the present application, the isolated antigen-binding protein may comprise L-FR4, and the L-FR4 may comprise an amino acid sequence as set forth in SEQ ID NO: 75: FGGGTKX₇EX₉K (SEQ ID NO: 75), where X₇ is L or V, and X₉ is I or V For example, it may be divided according to Chothia rules.

In some cases, compared with an amino acid sequence as set forth in SEQ ID NO: 14, the L-FR4 may comprise at least an amino acid substitution at a position selected from the group consisting of amino acid substitutions at X₇ and/or X₉.

For example, the L-FR4 may comprise an amino acid sequence as set forth in SEQ ID NO: 14 or 24.

In the present application, the isolated antigen-binding protein may comprise a light chain variable region VL, and the VL may comprise an amino acid sequence as set forth in SEQ ID NO: 67: DIX₃X₄TQSX₈X₉FX₁₁SX₁₃SVGDRVX₂₀ITCKASQDVGTAVAWYQQKPGX₄₂X₄₃PKLLIYW ASTRHTGVPX₆₀RFX₆₀GSGSGTX₇₀FTLTISX₇₇X₇₈QX₈₀EDX₈₃AX₈₅YFCQQYSSYPWTFG GGTKX₁₀₄EX₁₀₆K (SEQ ID NO: 67), where X₃ is Q or V, X₄ is L or M, X₈ is P or H, X₉ is K or S, X₁₁ is L or M, X₁₃ is A or T, and X₂₀ is S or T, X₄₂ is K or Q, X₄₃ is A or S, X₆₀ is D or S, X₆₃ is S or T, X₇₀ is D or E, X₇₇ is N or S, X₇₈ is L or V, X₈₀ is P or S, X₈₃ is F or L, and X₈₅ is D or T, X₁₀₄ is L or V, and X₁₀₆ is I or V For example, it may be divided according to Chothia rules.

In some cases, compared with an amino acid sequence as set forth in SEQ ID NO: 16, the VL may comprise at least an amino acid substitution at a position selected from the group consisting of X₃, X₄, X₈, X₉, X₁₁, X₁₃, X₂₀, X₄₂, X₄₃, X₆₀, X₆₃, X₇₀, X₇₇, X₇₈, X₈₀, X₈₃, X₈₅, X₁₀₄ and/or X₁₀₆.

For example, the VL may comprise an amino acid sequence as set forth in SEQ ID NO: 16 or 26.

In the present application, the isolated antigen-binding protein may comprise HCDR1, HCDR2, HCDR3, LCDR1, LCDR2 and LCDR3, and the HCDR1 may comprise an amino acid sequence as set forth in SEQ ID NO: 1, and the HCDR2 may comprise an amino acid sequence as set forth in SEQ ID NO: 2, the HCDR3 may comprise an amino acid sequence as set forth in SEQ ID NO: 3, the LCDR1 may comprise an amino acid sequence as set forth in SEQ ID NO: 8, the LCDR2 may comprise an amino acid sequence as set forth in SEQ ID NO: 9, and the LCDR3 may comprise an amino acid sequence as set forth in SEQ ID NO: 10.

In the present application, the isolated antigen-binding protein may comprise HCDR1, HCDR2, HCDR3, LCDR1, LCDR2 and LCDR3, and the HCDR1 may comprise an amino acid sequence as set forth in SEQ ID NO: 1, the HCDR2 may comprise an amino acid sequence as set forth in SEQ ID NO: 85, the HCDR3 may comprise an amino acid sequence as set forth in SEQ ID NO: 3, the LCDR1 may comprise an amino acid sequence as set forth in SEQ ID NO: 8, the LCDR2 may comprise an amino acid sequence as set forth in SEQ ID NO: 9, and the LCDR3 may comprise an amino acid sequence as set forth in SEQ ID NO: 10.

In the present application, the isolated antigen-binding protein may comprise VH and VL, the VH may comprise an amino acid sequence as set forth in SEQ ID NO: 65, and the VL may comprise an amino acid sequence as set forth in SEQ ID NO: 67.

In the present application, the isolated antigen-binding protein may comprise VH and VL, the VH may comprise an amino acid sequence as set forth in SEQ ID NO: 15 or 25, and the VL may comprise an amino acid sequence as set forth in SEQ ID NO: 16 or 26.

For example, the isolated antigen-binding protein may comprise VH and VL, the VH may comprise an amino acid sequence as set forth in SEQ ID NO: 15, and the VL may comprise an amino acid sequence as set forth in SEQ ID NO: 16.

For example, the isolated antigen-binding protein may comprise VH and VL, the VH may comprise an amino acid sequence as set forth in SEQ ID NO: 25, and the VL may comprise an amino acid sequence as set forth in SEQ ID NO: 26.

For example, the isolated antigen-binding protein may comprise VH and VL, the VH may comprise an amino acid sequence as set forth in SEQ ID NO: 86, and the VL may comprise an amino acid sequence as set forth in SEQ ID NO: 26.

In the present application, the isolated antigen-binding protein may comprise a heavy chain constant region, which may be derived from IgG. For example, the heavy chain constant region may be derived from human IgG. the heavy chain constant region may be derived from IgG1, IgG2, IgG3 and IgG4. For example, the heavy chain constant region may be derived from human IgG4. Compared with natural human IgG4, the heavy chain constant region may be amino acid mutated. For example, the heavy chain constant region may be a sequence obtained from performing S228P amino acid point mutation on IgG4. For example, the heavy chain constant region may comprise an amino acid sequence as set forth in SEQ ID NO: 77 or 81.

In the present application, the isolated antigen-binding protein may comprise a heavy chain, and the heavy chain may comprise an amino acid sequence as set forth in SEQ ID NO:79.

In the present application, the isolated antigen-binding protein may comprise a heavy chain, and the heavy chain may comprise an amino acid sequence as set forth in SEQ ID NO:87.

In the present application, the isolated antigen-binding protein may comprise a light chain constant region, and the light chain constant region may be derived from a light chain λ and a light chain κ. For example, the light chain constant region may comprise an amino acid sequence as set forth in SEQ ID NO: 78 or 82.

In the present application, the isolated antigen-binding protein may comprise a light chain, and the light chain may comprise an amino acid sequence as set forth in SEQ ID NO: 80 or 84.

For example, the isolated antigen-binding protein may comprise a heavy chain and a light chain, the heavy chain may comprise an amino acid sequence as set forth in SEQ ID NO: 79, and the light chain may comprise an amino acid sequence as set forth in SEQ ID NO: 80.

For example, the isolated antigen-binding protein may comprise a heavy chain and a light chain, the heavy chain may comprise an amino acid sequence as set forth in SEQ ID NO: 83, and the light chain may comprise an amino acid sequence as set forth in SEQ ID NO: 84.

For example, the isolated antigen-binding protein may comprise a heavy chain and a light chain, the heavy chain may comprise an amino acid sequence as set forth in SEQ ID NO: 87, and the light chain may comprise an amino acid sequence as set forth in SEQ ID NO: 84.

The isolated antigen-binding protein of the present application may comprise HCDR3, the HCDR3 may comprise CDR3 of VH with an amino acid sequence as set forth in SEQ ID NO: 66.

In the present application, the isolated antigen-binding protein may comprise HCDR3, and the HCDR3 may comprise CDR3 of VH with an amino acid sequence as set forth in SEQ ID NO: 41 or 51. For example, the isolated antigen-binding protein may comprise HCDR3, the HCDR3 may comprise an amino acid sequence as set forth in SEQ ID NO: 29.

In the present application, the isolated antigen-binding protein may comprise HCDR2, and the HCDR2 may comprise CDR2 of VH with an amino acid sequence as set forth in SEQ ID NO: 66.

In the present application, the isolated antigen-binding protein may comprise HCDR2, and the HCDR2 may comprise CDR2 of VH with an amino acid sequence as set forth in SEQ ID NO: 41 or 51. For example, the isolated antigen-binding protein may comprise HCDR2, and the HCDR2 may comprise an amino acid sequence as set forth in SEQ ID NO: 28.

In the present application, the isolated antigen-binding protein may comprise HCDR1, and the HCDR1 may comprise CDR1 of VH with an amino acid sequence as set forth in SEQ ID NO: 66.

In the present application, the isolated antigen-binding protein may comprise HCDR1, and the HCDR1 may comprise CDR1 of VH with an amino acid sequence as set forth in SEQ ID NO: 41 or 51. For example, the isolated antigen-binding protein may comprise HCDR1, and the HCDR1 may comprise an amino acid sequence as set forth in SEQ ID NO: 27.

In the present application, the isolated antigen-binding protein may comprise HCDR1, HCDR2 and HCDR3, the HCDR1 may comprise CDR1 of VH with an amino acid sequence as set forth in SEQ ID NO: 66, the HCDR2 may comprise CDR2 of VH with an amino acid sequence as set forth in SEQ ID NO: 66, and the HCDR3 may comprise CDR3 of VH with an amino acid sequence as set forth in SEQ ID NO: 66.

In the present application, the isolated antigen-binding protein may comprise HCDR1, HCDR2 and HCDR3, the HCDR1 may comprise CDR1 of VH with an amino acid sequence as set forth in SEQ ID NO: 41 or 51, the HCDR2 may comprise CDR2 of VH with an amino acid sequence as set forth in SEQ ID NO: 41 or 51, and the HCDR3 may comprise CDR3 of VH with an amino acid sequence as set forth in SEQ ID NO: 41 or 51.

For example, the isolated antigen-binding protein may comprise CDR1 of VH with an amino acid sequence as set forth in SEQ ID NO: 41, CDR2 of VH with an amino acid sequence as set forth in SEQ ID NO: 41, and CDR3 of VH with an amino acid sequence as set forth in SEQ ID NO: 41.

For example, the isolated antigen-binding protein may comprise CDR1 of VH with an amino acid sequence as set forth in SEQ ID NO: 51, CDR2 of VH with an amino acid sequence as set forth in SEQ ID NO: 51, and CDR3 of VH with an amino acid sequence as set forth in SEQ ID NO: 51.

For example, the isolated antigen-binding protein described in the present application may comprise HCDR1, HCDR2 and HCDR3, wherein the HCDR1 may comprise an amino acid sequence as set forth in SEQ ID NO: 27, the HCDR2 may comprise an amino acid sequence as set forth in SEQ ID NO: 28 and the HCDR3 may comprise an amino acid sequence as set forth in SEQ ID NO: 29.

In the present application, the isolated antigen-binding protein may comprise H-FR1, and the H-FR1 may comprise the amino acid sequence as set forth in SEQ ID NO: 58: QVQLX₅QSGAEX₁₁X₁₂X₁₃PGASVKX₂₀SCKAX₂₅GFTFI (SEQ ID NO: 58), where X₅ is V or Q, X₁₁ is L or V, X₁₂ is K or V, X₁₃ is K or R, X₂₀ is L or V, and X₂₅ is L or S. For example, it may be divided according to Chothia rules.

In some cases, compared with an amino acid sequence as set forth in SEQ ID NO: 30, the H-FR1 may comprise at least an amino acid substitution at a position selected from the group consisting of amino acid substitutions at X₅, X₁₁, X₁₂, X₁₃, X₂₀ and/or X₂₅.

For example, the H-FR1 may comprise an amino acid sequence as set forth in SEQ ID NO: 30 or 43.

In the present application, the isolated antigen-binding protein may comprise H-FR2, and the H-FR2 may comprise an amino acid sequence as set forth in SEQ ID NO: 60: WVRQX₅PX₇X₈GLEWIG (SEQ ID NO: 60), where X₅ is A or T, X₇ is V or G, and X₈ is H or Q. For example, it may be divided according to Chothia rules.

In some cases, compared with an amino acid sequence as set forth in SEQ ID NO: 31, the H-FR2 may comprise at least an amino acid substitution at a position selected from the group consisting of amino acid substitutions at X₅ and/or X₈.

For example, the H-FR2 may comprise an amino acid sequence as set forth in SEQ ID NO: 31 or 44.

In the present application, the isolated antigen-binding protein may comprise H-FR3, and the H-FR3 may comprise an amino acid sequence as set forth in SEQ ID NO: 62: X₁ATLX₅ADISX₁₀X₁₁TAX₁₄MEX₁₇SX₁₉LX₂₁SX₂₃DX₂₅AVYYCTR (SEQ ID NO: 62), where X₁ is K or R, X₅ is I or T, X₁₀ is T or I, X₁₁ is N or S, X₁₄ is S or Y, X₁₇ is L or V, X₁₉ is R or S, X₂₁ is R or T, X₂₃ is D or E, and X₂₅ is S or T. For example, it may be divided according to Chothia rules.

In some cases, compared with an amino acid sequence as set forth in SEQ ID NO: 32, the H-FR3 may comprise at least an amino acid substitution at a position selected from the group consisting of amino acid substitutions at X₁, X₅, X₁₀, X₁₁, X₁₄, X₁₇, X₁₉, X₂₁, X₂₃ and/or X₂₅.

For example, the H-FR3 may comprise an amino acid sequence as set forth in SEQ ID NO: 32 or 45.

In the present application, the isolated antigen-binding protein may comprise H-FR4, and the H-FR4 may comprise an amino acid sequence as set forth in SEQ ID NO: 64: WGX3GTTVTVSS (SEQ ID NO: 64), where X₃ is A or Q. For example, it may be divided according to Chothia rules.

In some cases, compared with an amino acid sequence as set forth in SEQ ID NO: 33, the H-FR4 may comprise at least an amino acid substitution at a position selected from the group consisting of an amino acid substitution at X₃.

For example, the H-FR4 may comprise an amino acid sequence as set forth in SEQ ID NO: 33 or 46.

In the present application, the isolated antigen-binding protein may comprise a heavy chain variable region VH, and the VH may comprise an amino acid sequence as set forth in SEQ ID NO: 66: QVQLX₅QSGAEX₁₁X₁₂X₁₃PGASVKX₂₀SCKAX₂₅GFTFIDYEMHWVRQX₄₀PX₄₂X₄₃GLE WIGGIHPGSGGTAYNQKFKGX₆₇ATLX₇₁ADISX₇₆X₇₇TAX₈ₒMEX₈₃SX₈₅LX₈₇SX₈₉DX₉₁AV YYCTREGFDVGWYFDVWGX₁₁₂GTTVTVSS (SEQ ID NO: 66), where X₅ is V or Q, X₁₁ is L or V, X₁₂ is K or V, X₁₃ is K or R, X₂₀ is L or V, X₂₅ is L or S, X₄₀ is A or T, X₄₂ is V or G, X₄₃ is H or Q, X₆₇ is K or R, X₇₁ is I or T, X₇₆ is T or I, X₇₇ is N or S, X₈₀ is S or Y, X₈₃ is L or V, X₈₅ is R or S, X₈₇ is R or T, X₈₉ is D or E, X₉₁ is S or T, and X₁₁₂ is A or Q. For example, it may be divided according to Chothia rules.

In some cases, compared with an amino acid sequence as set forth in SEQ ID NO: 41, the VH may comprise at least an amino acid substitution at a position selected from the group consisting of amino acid substitutions at X₅, X₁₁, X₁₂, X₁₃, X₂₀, X₂₅, X₄₀, X₄₂, X₄₃, X₆₇, X₇₁, X₇₆, X₇₇, X₈₀, X₈₃, X₈₅, X₈₇, X₈₉, X₉₁ and/or X₁₁₂.

For example, the VH may comprise an amino acid sequence as set forth in SEQ ID NO: 41 or 51.

The isolated antigen-binding protein of the present application may comprise LCDR3, and the LCDR3 may comprise CDR3 of VL with an amino acid sequence as set forth in SEQ ID NO: 68.

In the present application, the isolated antigen-binding protein may comprise LCDR3, and the LCDR3may comprise CDR3 of VL with an amino acid sequence as set forth in SEQ ID NO: 52 or 42. For example, the isolated antigen-binding protein may comprise LCDR3, and the LCDR3 may comprise an amino acid sequence as set forth in SEQ ID NO: 36.

In the present application, the isolated antigen-binding protein may comprise LCDR2, and the LCDR2 may comprise CDR2 of VL with an amino acid sequence as set forth in SEQ ID NO: 68.

In the present application, the isolated antigen-binding protein may comprise LCDR2, and the LCDR2 may comprise CDR2 of VL with an amino acid sequence as set forth in SEQ ID NO: 52 or 42. For example, the isolated antigen-binding protein may comprise LCDR2, and the LCDR2 may comprise an amino acid sequence as set forth in SEQ ID NO: 35.

In the present application, the isolated antigen-binding protein may comprise LCDR1, and the LCDR1 may comprise CDR1 of VL with an amino acid sequence as set forth in SEQ ID NO: 68.

In the present application, the isolated antigen-binding protein may comprise LCDR1, and the LCDR1 may comprise CDR1 of VL with an amino acid sequence as set forth in SEQ ID NO: 52 or 42. For example, the isolated antigen-binding protein may comprise LCDR1, the LCDR1 may comprise an amino acid sequence as set forth in SEQ ID NO: 34.

For example, the isolated antigen-binding proteins described in the present application may comprise LCDR1, LCDR2 and LCDR3, wherein the LCDR1 may comprise an amino acid sequence as set forth in SEQ ID NO: 34, the LCDR2 may comprise an amino acid sequence as set forth in SEQ ID NO: 35, and the LCDR3 may comprise an amino acid sequence as set forth in SEQ ID NO: 36.

In the present application, the isolated antigen-binding protein may comprise L-FR1, and the L-FR1 may comprise an amino acid sequence as set forth in SEQ ID NO: 70: DVX₃MTQX₇PLSLPVX₁₄LGX₁₇X₁₈ASISC (SEQ ID NO: 70), where X₃ is L or V, X₇ is S or T, X₁₄ is S or T, X₁₇ is D or Q, and X₁₈ is Q or P. For example, it may be divided according to Chothia rules.

In some cases, compared with an amino acid sequence as set forth in SEQ ID NO: 37, the L-FR1 may comprise at least an amino acid substitution at a position selected from the group consisting of amino acid substitutions at X₃, X₇, X₁₄, X₁₇ and/or X₁₈.

For example, the L-FR1 may comprise an amino acid sequence as set forth in SEQ ID NO: 37 or 47.

In the present application, the isolated antigen-binding protein may comprise L-FR2, and the L-FR2 may comprise an amino acid sequence as set forth in SEQ ID NO: 72: WYX₃QX₅PGQSPX₁₁LLIY (SEQ ID NO: 72), where X₃ is L or Q, X₅ is K or R, and X₁₁ is K or R. For example, it may be divided according to Chothia rules.

In some cases, compared with an amino acid sequence as set forth in SEQ ID NO: 38, the L-FR2 may comprise at least an amino acid substitution at a position selected from the group consisting of amino acid substitutions at X₃, X₅ and/or X₁₁.

For example, L-FR2 may comprise an amino acid sequence as set forth in SEQ ID NO: 38 or 48.

In the present application, the isolated antigen-binding protein may comprise L-FR3, and the L-FR3 may comprise an amino acid sequence as set forth in SEQ ID NO: 74: GVPDRFSGSGSGTDFTLKISRVEX₂₄EDX₂₇GVYYC (SEQ ID NO: 74), where X₂₄ is A or Q, and X₂₇ is L or V For example, it may be divided according to Chothia rules.

In some cases, compared with an amino acid sequence as set forth in SEQ ID NO: 39, the L-FR3 may comprise at least an amino acid substitution at a position selected from the group consisting of amino acid substitutions at X₂₄ and/or X₂₇.

For example, the L-FR3 may comprise an amino acid sequence as set forth in SEQ ID NO: 39 or 49.

In the present application, the isolated antigen-binding protein may comprise L-FR4, and the L-FR4 may comprise an amino acid sequence as set forth in SEQ ID NO: 76: FGX₃GTKLEIK (SEQ ID NO: 76), where X₃ is G or Q. For example, it may be divided according to Chothia rules.

In some cases, compared with an amino acid sequence as set forth in SEQ ID NO: 40, the L-FR4 may comprise at least an amino acid substitution at X₃.

For example, the L-FR4 may comprise an amino acid sequence as set forth in SEQ ID NO: 40 or 50.

In the present application, the isolated antigen-binding protein may comprise a light chain variable region VL, and the VL may comprise an amino acid sequence as set forth in SEQ ID NO: 68: DVX₃MTQX₇PLSLPVX₁₄LGX₁₇X₁₈ASISCRSSQSIVHSDGDTFLEWYX₄₂QX₄₄PGQSPX₅₀ LLIYKVSNRFSGVPDRFSGSGSGTDFTLKISRVEX₈₅EDX₈₈GVYYCFQGSHVPYTFGX₁₀₅ GTKLEIK (SEQ ID NO: 68), where X₃ is L or V, X₇ is S or T, X₁₄ is S or T, X₁₇ is D or Q, X₁₈ is Q or P, X₄₂ is L or Q, X₄₄ is K or R, and X₅₀ is K or R, X₈₅ is A or Q, X₈₈ is L or V, and X₁₀₅ is G or Q. For example, it may be divided according to Chothia rules.

In some cases, compared with an amino acid sequence as set forth in SEQ ID NO: 42, the VL may comprise at least an amino acid substitution at a position selected from the group consisting of amino acid substitutions at X₃, X₇, X₁₄, X₁₇, X₁₈, X₄₂, X₄₄, X₅₀, X₈₅, X₈₈ and/or X₁₀₅.

For example, the VL may comprise an amino acid sequence as set forth in SEQ ID NO: 42 or 52.

In the present application, the isolated antigen-binding protein may comprise HCDR1, HCDR2, HCDR3, LCDR1, LCDR2 and LCDR3, and the HCDR1 may comprise an amino acid sequence as set forth in SEQ ID NO: 27, and the HCDR2 may comprise an amino acid sequence as set forth in SEQ ID NO: 28. the HCDR3 may comprise an amino acid sequence as set forth in SEQ ID NO: 29, the LCDR1 may comprise an amino acid sequence as set forth in SEQ ID NO: 34, the LCDR2 may comprise an amino acid sequence as set forth in SEQ ID NO: 35, and the LCDR3 may comprise an amino acid sequence as set forth in SEQ ID NO: 36.

In the present application, the isolated antigen-binding protein may comprise VH and VL, and the VH may comprise an amino acid sequence as set forth in SEQ ID NO: 66, and the VL may comprise an amino acid sequence as set forth in SEQ ID NO: 68.

In the present application, the isolated antigen-binding protein may comprise VH and VL, and the VH may comprise an amino acid sequence as set forth in SEQ ID NO: 41 or 51, and the VL may comprise an amino acid sequence as set forth in SEQ ID NO: 42 or 52.

For example, the isolated antigen-binding protein may comprise VH and VL, and the VH may comprise an amino acid sequence as set forth in SEQ ID NO: 41, and the VL may comprise an amino acid sequence as set forth in SEQ ID NO: 42.

For example, the isolated antigen-binding protein may comprise VH and VL, and the VH may comprise an amino acid sequence as set forth in SEQ ID NO: 51, and the VL may comprise an amino acid sequence as set forth in SEQ ID NO:52.

In the present application, the isolated antigen-binding protein may comprise a heavy chain constant region, and the heavy chain constant region may be derived from IgG. For example, the heavy chain constant region may be derived from human IgG. the heavy chain constant region may be derived from IgG1, IgG2, IgG3 and IgG4. For example, the heavy chain constant region may be derived from human IgG4. Compared with natural human IgG4, the heavy chain constant region may be amino acid mutated. For example, the heavy chain constant region may be a sequence obtained from performing S228P amino acid point mutation on IgG4. For example, the heavy chain constant region may comprise an amino acid sequence as set forth in SEQ ID NO: 77 or 81.

In the present application, the isolated antigen-binding protein may comprise a heavy chain, and the heavy chain may comprise an amino acid sequence as set forth in SEQ ID NO:79.

In the present application, the isolated antigen-binding protein may comprise a heavy chain, the heavy chain may comprise an amino acid sequence as set forth in SEQ ID NO:87.

In the present application, the isolated antigen-binding protein may comprise a light chain constant region, and the light chain constant region may be derived from a light chain λ and a light chain κ. For example, the light chain constant region may comprise an amino acid sequence as set forth in SEQ ID NO: 78 or 82.

In the present application, the isolated antigen-binding protein may comprise a light chain, and the light chain may comprise an amino acid sequence as set forth in SEQ ID NO: 80 or 84.

For example, the isolated antigen-binding protein may comprise a heavy chain and a light chain, the heavy chain may comprise an amino acid sequence as set forth in SEQ ID NO: 79, and the light chain may comprise an amino acid sequence as set forth in SEQ ID NO: 80.

For example, the isolated antigen-binding protein may comprise a heavy chain and a light chain, the heavy chain may comprise an amino acid sequence as set forth in SEQ ID NO: 83, and the light chain may comprise an amino acid sequence as set forth in SEQ ID NO: 84.

For example, the isolated antigen-binding protein may comprise a heavy chain and a light chain, the heavy chain may comprise an amino acid sequence as set forth in SEQ ID NO: 87, and the light chain may comprise an amino acid sequence as set forth in SEQ ID NO: 84.

In the present application, a part of the amino acid sequence of each heavy chain or light chain of the antigen-binding protein is homologous to the corresponding amino acid sequence in an antibody from a specific species, or belongs to a specific category. For example, the variable region and constant portion of light chain and heavy chain are both from the variable region and constant region of antibody of one animal species (such as human). In the present application, the homologue may be a protein and/or a polypeptide having at least about 85% (for example, at least about 85%, about 90%, about 91%, about 92%, about 93%, about 94%, about 95%, about 96%, about 97%, about 98%, about 99% or more) identity with the amino acid sequence of the protein and/or the polypeptide (for example, an antibody or fragment thereof that specifically binds to PD-L1 protein).

In the present application, the homology generally refers to similarity, likeness or association between two or more sequences. The alignment for determining the percentage of sequence homology can be realized in various ways known in the art, for example, using publicly available computer softwares such as BLAST, BLAST-2, ALIGN or Megalign (DNASTAR) softwares. Those skilled in the art may determine appropriate parameters for alignment sequences, including any algorithm needed to achieve maximum alignment within the full-length sequence being compared or within the target sequence area. The homology may also be determined by the following methods: FASTA and BLAST. For the description of FASTA algorithm, please refer to W. R. Pearson and D. J. Lipman, "Improved Tools for Biological Sequence Comparison", Proc. Natl. Acad. Sci., 85:2444-2448, 1988; and D. J. Lipman and W. R. Pearson, " Rapid and sensitive protein similarity searches", Science, 227:1435-1441, 1989. Descriptions of BLAST algorithm may be found in S. Altschul, W. Gish, W. Miller, E. W. Myers and D. Lipman, "Basic Local Alignment Search Tool", Journal of Molecular Biology, 215:403-410, 1990.

### Test Method

The physical/chemical properties and/or biological activities of the PD-1 antigen-binding protein described in the present application may be identified, screened or characterized by various assays known in the art.

In one aspect, for example, the antigen binding activity of the antigen-binding protein of the present application may be tested by known methods such as enzyme-linked immunosorbent assay (ELISA), western blot (e.g., protein blot), flow cytometry (e.g., FACS), immunohistochemistry, immunofluorescence or the like.

### Nucleic acid, vector, host cell and preparation method

In another aspect, the present application also provides one or more isolated nucleic acid molecules. The one or more nucleic acid molecules may encode the antigen-binding protein described herein. For example, each nucleic acid molecule in the one or more nucleic acid molecules may encode the complete antigen-binding protein, or may encode a portion thereof (for example, one or more of HCDR1-3, LCDR1-3, VL, VH, a light chain or a heavy chain).

The nucleic acid molecule described in the present application may be isolated. For example, it may be produced or synthesized by the following methods: (i) amplification *in vitro,* for example, polymerase chain reaction (PCR) amplification, (ii) cloning and recombination, (iii) purification, for example, fractionation by enzyme digestion and gel electrophoresis, or (iv) synthesis, for example, chemical synthesis. In some embodiments, the isolated nucleic acid is a nucleic acid molecule prepared by recombinant DNA technology.

In the present application, the nucleic acid encoding the antibody and antigen-binding fragment thereof may be prepared by various methods known in the art, including but not limited to restriction fragment manipulation or overlapping extension PCR using synthetic oligonucleotides.

In another aspect, the present application provides one or more vectors comprising one or more nucleic acid molecules described herein. Each vector may comprise one or more of the nucleic acid molecules. In addition, the vector may also comprise other genes, such as marker genes that allow selection of the vector in appropriate host cells and under appropriate conditions. In addition, the vector may also comprise an expression control element that allows the coding region to be correctly expressed in an appropriate host. For example, the vector is an expression vector.

In another aspect, the present application provides a host cell, and the host cell may comprise one or more nucleic acid molecules and/or one or more vectors described herein. In some embodiments, each or every host cell may comprise one or more nucleic acid molecules or vectors described herein. In some embodiments, each or every host cell may comprise a plurality (e.g., two or more) or a variety (e.g., two or more kinds) of nucleic acid molecules or vectors described herein.

In another aspect, the present application provides a method for preparing the antibody or antigen-binding fragment thereof. The method may comprise culturing the host cell described in the present application under conditions such that the antibody or antigen-binding fragment thereof is expressed. For example, with suitable culture medium, suitable temperature and culture time, etc., these methods are known to those skilled in the art.

### Pharmaceutical composition. Method and Use

In another aspect, the present application provides a pharmaceutical composition. The pharmaceutical composition may comprise the antigen-binding protein, the polypeptide, the nucleic acid molecule, the vector, the host cell described herein, and optionally a pharmaceutically acceptable carrier. The pharmaceutically acceptable adjuvant is not toxic to the recipient at the dosage and concentration adopted, and the pharmaceutical composition in the present application may also comprise more than one active compounds, which are those active compounds with complementary activities and usually do not adversely affect each other. The form and effective amount of such pharmaceuticals depend on, for example, the amount and form of antagonists present in the preparation, and the clinical parameters of the subject.

The pharmaceutical composition may be used for inhibiting tumor growth. For example, the pharmaceutical composition of the present application may inhibit or delay the development or progression of a disease, reduce the tumor size (even basically eliminate the tumor), and/or alleviate and/or stabilize the disease conditions.

The pharmaceutical composition of the present application may comprise a prophylactically and/or therapeutically effective amount of the antibody, antigen-binding fragment thereof. The prophylactically and/or therapeutically effective amount is the dosage that is required to prevent and/or treat (at least partially treat) a disease or disorder and/or any complications thereof in a subject having or at risk of developing the disease or disorder.

In another aspect, the present application provides the use of the antigen-binding protein and/or the fusion protein in the manufacture of a medicament. The medicament is used for treating cancer, inhibiting tumor growth and/or inhibiting tumor cell proliferation. In some embodiments, the tumor or cancer is a tumor or cancer with abnormal expression of PD-L1 and/or PD-1. In some embodiments, the tumor comprises a tumor with a high expression of PD-1 or PD-L1. In some embodiments, the tumor comprises a solid tumor and/or a non-solid tumor. In some embodiments, the tumor comprises melanoma, lung cancer, renal cancer, esophageal cancer, head and neck cancer, lymphoma, liver cancer and/or gastric cancer.

In another aspect, the present application provides a method of inhibiting the binding of PD-L1 to PD-1, which comprises administering the antigen-binding protein and/or the polypeptide described herein. For example, the method may be *ex vivo* or *in vitro.* For example, the method may be a method for non-therapeutic purpose. In some cases, the method may comprise contacting a biological sample with the antigen-binding protein and/or PD-1 described herein under conditions that allow the antigen-binding protein and/or PD-1 to bind to PD-L1, detecting whether a complex is formed between the antigen-binding protein and PD-L1, and detecting whether a complex is formed between PD-1 and PD-L1.

In another aspect, the present application provides a method of inhibiting the binding of PD-L2 to PD-1, which comprises administering the antigen-binding protein and/or the polypeptide described herein. For example, the method may be *ex vivo* or *in vitro.* For example, the method may be a method for non-therapeutic purpose. In some cases, the method may comprise contacting a biological sample with an antigen-binding protein and/or PD-1 described herein under conditions that allow binding of the antigen-binding protein and/or PD-1 to PD-L2, detecting whether a complex is formed between the antigen-binding protein and PD-L2, and detecting whether a complex is formed between PD-1 and PD-L2.

In another aspect, the present application provides a method for stimulating the secretion of cytokines in immune cells, which comprises administering the isolated antigen-binding protein and/or the polypeptide. The cytokine may be IL-2. The immune cells may be lymphocytes. For example, T lymphocytes. For example, the method may be *ex vivo* or *in vitro.* For example, the method may be a method for non-therapeutic purpose.

In another aspect, the present application provides a method for detecting the presence and/or content of PD-1 protein, which comprises administering the isolated antigen-binding protein and/or the polypeptide. For example, the method may be *ex vivo* or *in vitro.* For example, the method may be a method for non-therapeutic purpose.

The present application also provides the use of antigen-binding protein in a method for diagnosing a subject suffering from a tumor or cancer, the method comprises: determining the presence or expression level of PD-1 in a sample obtained from the subject by contacting the sample with the antigen-binding protein of the present application and detecting the presence of the bound antibody.

In another aspect, the present application provides a chimeric antigen receptor (CAR), which may comprise the nucleic acid molecule or antigen-binding protein described in the present application.

In another aspect, the present application provides an antibody drug conjugate, which may comprise a cytotoxic agent and an antigen-binding fragment described in the present application. An antibody-drug conjugate generally refers to linking an antibody to a small molecular cytotoxic drug by using a specific linker, and its main components may comprise an antibody, a linker and a small molecular cytotoxic drug.

In another aspect, the present application provides a kit, which may comprise the antigen-binding protein, chimeric antigen receptor, genetically modified cell, antibody drug conjugate described in the present application, and/or the pharmaceutical composition described in the present application. It may comprise the antigen-binding protein, chimeric antigen receptor, genetically modified cell and/or antibody drug conjugate described in the present application in a single common container, and may also be optionally combined with one or more therapeutic agents, optionally formulated together in pharmaceutical compositions.

In another aspect, the present application provides a drug delivery device, which can be used for administering the antigen-binding protein or a pharmaceutical composition thereof.

Without to be bound by any theory, the following examples are only for illustrating the fusion protein, preparation method and use of the present application etc., and they are not used to limit the scope of the present application.

### Example

### Example 1. Preparation and Detection of Anti-PD-1 Humanized Antibody

### 1.1 Discovery of Hybridoma Antibody

Five Balb/c mice and five SJL mice were immunized with Human PD-1, Mouse IgG2a Fc Tag fusion protein (Acrobiosystem, PD1-H5255a) and pcDNA3.4-hPD-1 plasmid DNA, with an interval of 2 to 3 weeks each time. After three times, booster immunization was performed with Human PD-1, Mouse IgG2a Fc Tag fusion protein or CHOK1-hPD-1 stably transformed cell line (GenScript, M00529) for 1 to 3 times, and blood was taken to determine the titer. The titer was determined by determining the binding of serum to CHOK1 or 293T cells overexpressing human PD-1 protein using flow cytometer, and mouse spleen cells with high binding titer were fused with myeloma (Sp2/0) cells.

Clones that specifically bind to CHOK1-hPD-1 cells and can simultaneously block the binding of ligands PD-L1 and PD-L2 to CHOK1-hPD1 cells were screened from the fusion plate by flow cytometer, subcloning was carried out by limiting dilution assay, and finally a monoclonal that can secrete specific antibodies was screened. Monoclonal antibodies were obtained by small-scale production and purification in serum-free medium for subsequent identification, including the detection of the binding ability of antibodies to CHOK1-hPD1, the function of blocking binding of PD-L1, PD-L2 to CHOK1-hPD-1, and the mixed lymphocyte experiment to finally obtain candidate anti-human PD-1 monoclonal antibodies 41D2-2C3D7 and 46H3A8. The variable region sequences of murine hybridoma clones 41D2-2C3D7 and 46H3A8 obtained by monoclonal antibody sequencing are as follows:
> 41D2-2C3D7 heavy chain variable region sequence 
EVKLVESGGGLVKPGGSLKLACAASGFTFSNYDMSWVRQTPEKRLEWVATISGG
GSYTYYPDSVKGRFTISRDNAKNNLYLQMSSLRSEDTALYYCVSPYYGMEYWGQGT
SVTVSS) (SEQ ID NO: 15)
>41D2-2C3D7 light chain variable region sequence 
DIVMTQSHKFMSTSVGDRVSITCKASQDVGTAVAWYQQKPGQSPKLLIYWASTR
HTGVPDRFTGSGSGTDFTLTISNVQSEDLADYFCQQYSSYPWTFGGGTKLEVK (SEQ
ID NO: 16)
>46H3A8 heavy chain variable region sequence 
QVQLQQSGAELVRPGASVKLSCKALGFTFIDYEMHWVRQTPVHGLEWIGGIHPG
SGGTAYNQKFKGKATLIADISTNTASMEVSSLTSEDSAVYYCTREGFDVGWYFDVWG
AGTTVTVSS (SEQ ID NO: 41)
>46H3A8 light chain variable region sequence

DVLMTQTPLSLPVSLGDQASISCRSSQSIVHSDGDTFLEWYLQKPGQSPKLLIYK
VSNRFSGVPDRFSGSGSGTDFTLKISRVEGEDLGVYYCFQGSHVPYTFGGGTKLEIK
(SEQ ID NO: 42)

**Table 1: Heavy Chain and Light Chain CDR Sequences of Murine Hybridoma Clone 41D2-2C3D7**

| Heavy Chain | | Light Chain | |
|---|---|---|---|
| HCDR1 | GFTFSNY (SEQ ID NO: 1) | LCDR1 | KASQDVGTAVA (SEQ ID NO: 8) |
| HCDR2 | SGGGSY (SEQ ID NO: 2) | LCDR2 | WASTRHT (SEQ ID NO: 9) |
| HCDR3 | PYYGMEY (SEQ ID NO: 3) | LCDR3 | QQYSSYPWT (SEQ ID NO: 10) |

**Table 2: Heavy Chain and Light Chain CDR Sequences of Murine Hybridoma Clone 46H3A8**

| Heavy Chain | | Light Chain | |
|---|---|---|---|
| HCDR1 | DYEMH (SEQ ID NO: 27) | LCDR1 | RSSQSIVHSDGDTFLE (SEQ ID NO: 34) |
| HCDR2 | GIHPGSGGTAYNQKFKG (SEQ ID NO: 28) | LCDR2 | KVSNRFS (SEQ ID NO: 35) |
| HCDR3 | EGFDVGWYFDV (SEQ ID NO: 29) | LCDR3 | FQGSHVPYT (SEQ ID NO: 36) |

### Example 2. Humanization of Monoclonal Antibodies

### 2.1 Humanization of murine hybridoma antibody

The most homologous human Germline antibody (data source: IMGT) was selected as the humanized design framework (IGKV1-9*01, IGKJ4*01 were used as framework of the light chain, and IGHV3-21*01, IGHJ5*01 were used as framework of heavy chain) by a sequence alignment, and the variable regions of antibody light and heavy chains were numbered by Chothia [Chothia & Lesk, 1987], and antibody CDR regions were defined as follows: LCDR1(L24-L34), LCDR2(L50-L56), LCDR3(L89-L97), HCDR1(H26-H32), HCDR2(H52-H56) and HCDR3(H95-H97); humanized mutation of amino acids in light and heavy chain variable regions of antibodies was carried out according to sequence alignment and structural information of variable regions; expression vectors were designed, genes were synthesized, recombinant antibodies were expressed in mammalian cells and purified, the differences of humanized antibodies and chimeric antibodies in activities as well as physical and chemical properties were compared, and 1-2 rounds of humanization optimization were carried out.

Germline antibody sequence information:
IGKV1-9*01 
DIQLTQSPSFLSASVGDRVTITCRASQGISSYLAWYQQKPGKAPKLLIYAASTLQS
GVPSRFSGSGSGTEFTLTISSLQPEDFATYYCQQLNSYP (SEQ ID NO: 53)
IGKJ4*01
   LTFGGGTKVEIK (SEQ ID NO: 54)
IGHV3-21*01 
EVQLVESGGGLVKPGGSLRLSCAASGFTFSSYSMNWVRQAPGKGLEWVSSISSSS
SYIYYADSVKGRFTISRDNAKNSLYLQMNSLRAEDTAVYYCAR (SEQ ID NO: 55)
IGHJ5*01
   NWFDSWGQGTLVTVSS (SEQ ID NO: 56)

The following optimized designs of light and heavy chains are humanized sequences transplanted based on the above Germanline antibody as framework CDR (41D2HzL0 and 41D2HzH0 are the light and heavy chains of the chimeric antibody, and 41D2HzL4 and 41D2HzH3 are the light and heavy chains of the humanized antibody).
41D2HzL0 light chain variable region sequence: 
DIVMTQSHKFMSTSVGDRVSITCKASQDVGTAVAWYQQKPGQSPKLLIYWASTR
HTGVPDRFTGSGSGTDFTLTISNVQSEDLADYFCQQYSSYPWTFGGGTKLEVK (SEQ
ID NO: 16)
41D2HzL4 light chain variable region sequence: 
DIQLTQSPSFLSASVGDRVTITCKASQDVGTAVAWYQQKPGKAPKLLIYWASTRH
TGVPSRFSGSGSGTEFTLTISSLQPEDFATYFCQQYSSYPWTFGGGTKVEIK (SEQ ID
NO: 26)
41D2HzH0 heavy chain variable region sequence: 
EVKLVESGGGLVKPGGSLKLACAASGFTFSNYDMSWVRQTPEKRLEWVATISGG
GSYTYYPDSVKGRFTISRDNAKNNLYLQMSSLRSEDTALYYCVSPYYGMEYWGQGT
SVTVSS (SEQ ID NO: 15)
41D2HzH3 heavy chain variable region sequence:

EVQLVESGGGLVKPGGSLRLSCAASGFTFSNYDMSWVRQAPGKGLEWVSTISGG
GSYTYYADSVKGRFTISRDNAKNSLYLQMNSLRAEDTAVYYCVSPYYGMEYWGQGT
LVTVSS (SEQ ID NO: 25)

### 2.2 Humanization of murine hybridoma antibody 46H3A8

The humanization scheme of 46H3A8 is the same as 1.2.1, and the following optimized design of light and heavy chains are humanized sequences transplanted based on the Germline antibody as framework CDR (1910h3hzL0 and 1910h3hzH0 are the light and heavy chains of the chimeric antibody, and 1910h3hzL3 and 1910h3hzH4 are the light and heavy chains of the humanized antibody).
1910h3hzL0 light chain variable region sequence: 
DVLMTQTPLSLPVSLGDQASISCRSSQSIVHSDGDTFLEWYLQKPGQSPKLLIYK
VSNRFSGVPDRFSGSGSGTDFTLKISRVEGEDLGVYYCFQGSHVPYTFGGGTKLEIK
(SEQ ID NO: 42)
1910h3hzH0 heavy chain variable region sequence: 
QVQLQQSGAELVRPGASVKLSCKALGFTFIDYEMHWVRQTPVHGLEWIGGIHPG
SGGTAYNQKFKGKATLIADISTNTASMEVSSLTSEDSAVYYCTREGFDVGWYFDVWG
AGTTVTVSS (SEQ ID NO: 41)
1910h3hzL3 light chain variable region sequence: 
DVVMTQSPLSLPVTLGQPASISCRSSQSIVHSDGDTFLEWYQQRPGQSPRLLIYKV
SNRFSGVPDRFSGSGSGTDFTLKISRVEAEDVGVYYCFQGSHVPYTFGQGTKLEIK
(SEQ ID NO: 52)
1910h3hzH4 heavy chain variable region sequence:

QVQLVQSGAEVKKPGASVKVSCKASGFTFIDYEMHWVRQAPGQGLEWIGGIHP
GSGGTAYNQKFKGRATLTADISISTAYMELSRLRSDDTAVYYCTREGFDVGWYFDVW
GQGTTVTVSS (SEQ ID NO: 51)

### Example 3. Determination of Kinetic Parameters of humanized antibody

(1) The affinities of humanized antibodies 1910h3hzL3H4 and 41D2HzL4H3 with human PD-1 (Sino Biological, Catalog Number: 10377-H08H) were determined by Octet RED96e (Fortebio), and both antigens and antibodies were diluted by 1xPBST (1xPBS: Sangon, B548117-0500; 0.02% Tween 20: Sigma-Alorich, P1379), the concentration of the antigens was 100 nM, and the concentration of antibodies was 50 nM.
(2) Detection of samples on the instrument (Octet Data Acquisition 11.1.0.11): First, the sample was added into a 96-well plate (Greiner bio-one, 655209) with a system of 200 µL/well. Then the software parameters were set, the plate temperature was set to 30°C, and the frequency of collecting standard dynamic signals was 5.0 HZ. Next, the AHC sensor (Fortébio, Catalog Number: 18-0015) was pre-wetted with 1xPBST for 10 minutes, and then the sample was detected on the instrument. Each cycle incluedes the following steps: 1) immersing in buffer for 60 seconds; 2) detecting whether the antigen nonspecifically binding to the sensor; 3) regeneration of 10 mM glycine solution with pH 1.7; 4) immersing in the buffer for 60 seconds; 5) immobilizing the antibody on the sensor for 20 seconds; 6) immersing the sensor in the buffer for 180 seconds; 7) the antigen binding to the antibody for 180 seconds; 8) dissociating the antigen from the antibody for 10 minutes; 9) regenerating the sensor.
(3) Data analysis

The association rate (Ka) and dissociation rate (Kd) of the antigen-antibody at a ratio of 1:1 were measured by Data Analysis 11.0 software of Fortebio, and the equilibrium dissociation constant (KD) of the antibody was calculated. The results are as follows:

**Table 3: Affinity of Humanized Antibody with Human PD-1**

| Candidate Antibody | K_{D} (M) | kon (1/Ms) | kdis (1/s) |
|---|---|---|---|
| 1910h3hzL3H4 | 8.038E-09 | 1.47E+05 | 1.18E-03 |
| 41D2HzL4H3 | 1.919E-09 | 2.83E+05 | 5.42E-04 |
| Pembrolizumab | 7.693E-09 | 5.45E+05 | 4.19E-03 |

From Table 3 it was concluded that the antibody 1910h3hzL3H4 had comparable affinity to human PD-1, compared with Pembrolizumab. The affinity of 41D2HzL4H3 to human PD-1 was higher than Pembrolizumab by 4.2 times. It is shown that the antigen-binding protein of the present application had higher binding affinity to PD-1.

### Example 4. Binding Activity of the Antigen-binding protein of the present application to Human or cynomolgus monkey PD-1 on the Cell Surface.

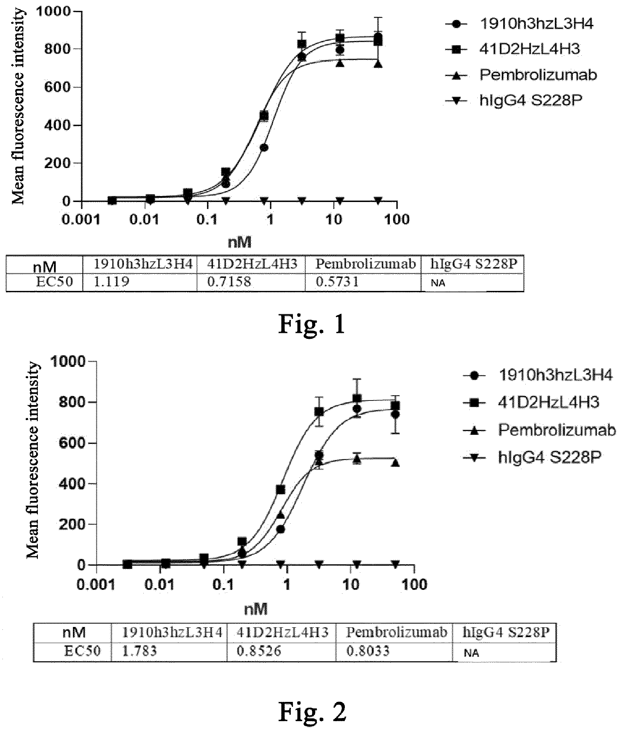

CHOK-hPD-1 (GenScript, M00529) cells or CHOK1-cynoPD-1 (GenScript, M00572) were collected, washed with FACS buffer (2% FBS in PBS) once, resuspended, and separated into a 96-well plate (Corning 3799) by 1E5/50ul/well. The antibody was added to the 96-well U-shaped plate, 50ul/ well, and incubated at 4°C for 1 hour. The mixture was centrifuged at 2000rpm for 5 minutes and the supernatant was removed, then 200ul FACS buffer was added into each well for washing once. Alexa Fluor 488 secondary antibody (Invitrogen) with a ratio of 1 : 1000 was added into each well, 100ul/well, mixed uniformly and incubated at 4°C for 1 hour. After centrifugation at 2000rpm for 5 minutes, the supernatant was removed, 200ul FACS buffer was added into each well for washing once, then 30ul FACS buffer was added for resuspension and detection on the instrument. The results of the experiment were shown in Fig. 1 and Fig. 2 below. Fig. 1 shows that the antigen-binding proteins 1910h3hzL3H4 and 41D2HzL4H3 described in the present application bind to the human PD-1 protein on the surface of CHOK1 cell. Fig. 2 shows that the antigen-binding proteins 1910h3hzL3H4 and 41D2HzL4H3 described in the present application bind to the cynomolgus monkey PD-1 protein on the surface of CHOK1 cell. The results show that the binding activities of 1910h3hzL3H4 and 41D2HzL4H3 to human or cynomolgus monkey PD-1 on the cell surface are comparable to Pembrolizumab.

### Example 5. The Antigen-binding protein of the Present Application Blocks the Binding Activity of Human PD-1 to PD-L1 or PD-L2 on the Cell Surface.

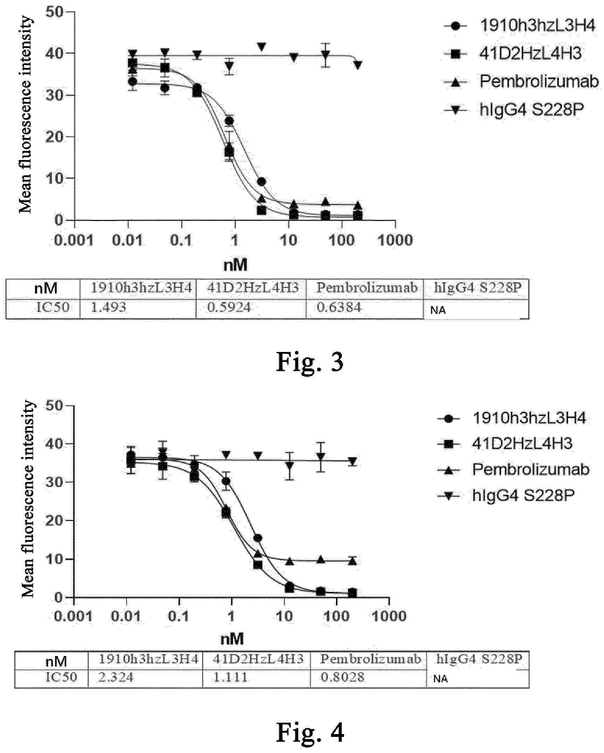

CHOK1-hPD-1 cells were collected, washed once with FACS buffer, resuspended, and separated into a 96-well U-shaped plate by 1E5/50ul/ well. The antibody was diluted and added into the 96-well plate, 25ul/well, and biotinylated PD-L1 (Sinobiological, Catalog Number. 10084-H02H-B) (or PD-L2, Acrobiosystem, Catalog Number. PD2-H82F6) was diluted and added into the 96-well plate, 25 ul/well, mixed uniformly and incubated at 4°C for 1 hour. After a centrifugation at 2000rpm for 5 minutes, the supernatant was removed, and 200 ul FACS buffer was added into each well for washing once. SA-Alexa Fluor 488 (Invitrogen Cat: S32354) with a ratio of 1: 1000 was added into each well, 100ul/well, mixed uniformly and incubated at 4°C for 30 minutes. After centrifugation at 2000rpm for 5 minutes, the supernatant was removed, 200 ul FACS buffer was added into each well for washing once, then 30ul FACS buffer was added for resuspension and detection on the instrument. Fig. 3 shows that the antigen-binding proteins 1910h3hzL3H4 and 41D2HzL4H3 described in the present application block the binding of human PD-L1 to human PD-1 protein on the surface of CHOK1. Fig. 4 shows that the antigen-binding proteins 1910h3hzL3H4 and 41D2HzL4H3 described in the present application block the binding of human PD-L2 to human PD-1 protein on the surface of CHOK1 cell. The results show that the binding activity of human PD-1 to PD-L1 or PD-L2 on the cell surface was blocked by 1910h3hzL3H4 and 41D2HzL4H3, comparable to the control antibody Pembrolizumab. It shows that the antigen-binding protein in the present application has excellent activity in blocking the binding of PD-1 to PD-L1 or PD-L2.

### Example 6. Mixed Lymphocyte Reaction: Secretion of Cytokine IL-2

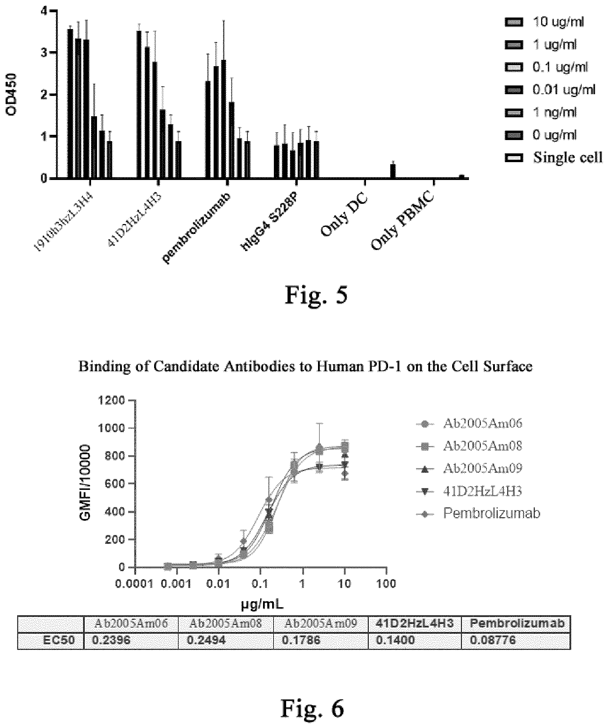

Human dendritic DC cells were resuscitated with cell culture medium (1640+ 2% FBS), the density of DC cells was adjusted to 1*10⁵ to 1*10⁷ cells /mL, then mitomycin C was added with a final concentration of 50 µg/ml, and after treated at 37 degree in the dark for 30 minutes 10 mL culture medium was added for termination; the mixture was centrifuged at 400g for 10 minutes, and then washed with 10 ml culture medium. Gradient dilution of anti-PD1 antibody: the greatest final concentration of antibody is 2.5 µg/mL (the preparation concentration is 10 µg/mL), and the gradient dilution is performed by ten-fold (5 concentration points + one 0 concentration), and then 50 µL of prepared anti-PD1 antibody was added into the corresponding cell culture plate (Corning, Catalog Number: 3599). DC cells treated with human peripheral blood lymphocytes PBMC and mitomycin C were collected, the density of DC cells was adjusted to 2*10⁵ cells/mL, and then the cells were added into the culture plate, with 50 µL/well, i.e., the number of DC cells per well was 1*10⁴ cells/well; the density of PBMC cells was adjusted to 2*10⁶ cells /mL, and then the cells were added into the culture plate, with 100 µL/well, i.e., the number of PBMC cells per well was 2*10⁵ cells/well. The cell culture plate was placed in a cell incubator with 5% carbon dioxide at 37°C for 5 days. After 5 days, the supernatant was collected followed by a centrifugation with 300g for 5 minutes, and content of IL-2 was detected with Human IL-2 ELISA Kit (BD, Catalog Number: 550611), the detection method is strictly performed according to the kit instructions, and the data were processed with GraphPad Prism software. The results were shown in Fig. 5. The results indicated that humanized antibody 1910h3hzL3H4 and 41D2HzL4H3 were able to stimulate lymphocytes to secrete IL-2, having similar activities to Pembrolizumab. It was indicated that the antigen-binding proteins of the present application had high abilities to stimulate immune cells to secrete cytokines.

### Example 7. Affinity Maturation of Antigen-binding proteins

### 7.1 Construction and Screening of Yeast Display Mutation Library, and Monoclonal Identification

### Construction and Screening of Yeast Display Mutation Library

Based on 41D2HzL4H3, the amino acids of the antigen-binding determinant (CDR) site were randomly mutated, constructing the mutation libraries of each CDR region. The sequences with robust antigen-specific-binding capacity were high-throughput screened by yeast display technology. The amino acid sequence of the heavy chain variable region of 41D2HzL4H3 was coded according to Chothia coding rules, and the CDR region was defined according to Chothia. For variable regions CDR1, CDR2 and CDR3 of each molecule, NNK mutation primers were designed to amplify the gene fragments of each CDR mutation library by polymerase chain reaction (PCR). Gene fragments of each CDR mutant library respectively in combination with yeast display plasmid were introduced into *Saccharomyces cerevisiae* strain EBY100 (purchased from ATCC), so that each CDR mutant library was displayed on the yeast surface in the form of Fab. At the same time, the parent sequence of 41D2HzL4H3 was displayed on the yeast surface in the form of Fab, which was used as a control.

After the library was cultured and induced, 1.5 ×10⁹ cells were taken from each library calculated based on 1 OD as 1.0×10⁷ cells, and the first round of enrichment was carried out by magnetic bead sorting system. The cells of Each library were resuspended in 1×PBSA (1×PBS+1%BSA) containing 5 nM biotin-labeled human PD-1 (Aero, Catalog Number: PD1-H82E4, abbreviated as Bitoin hPD-1) for 30 minutes; following being washed, Anti biotin beads (miltenyi, Catalog Number: 130-090-485) were added, mixed uniformly and incubated for 10 minutes, and then the positive cells were collected through a magnetic column (Quadro MACS Starting Kit). After the positive cells were cultured and induced again, 3.0×10⁷ cells were taken respectively, and the second round of flow sorting was carried out with 1 nM Bitoin hPD-1 to collect cell populations with high display level and strong binding ability to antigen. After the positive cells were cultured and induced again, 2.0×10⁷ cells were taken respectively, and the third round of flow sorting was carried out with 10 nM biotin-labeled monkey PD-1(Acro, Catalog Number: PD1-C82E6, abbreviated as Bitoin cyno PD-1). After sorting, the cells were taken for sequencing and analysis to obtain individual sequences with mutation in each CDR region of the heavy chain.

### Identification of Mutant Monoclonal Antibody

After sequencing analysis, 42 new mutant sequences were obtained, and the corresponding monoclonal antibodies were identified by flow cytometry staining; and they were stained by incubation with 1 nM Bitoin hPD-1 and 1 nM Bitoin cyno PD-1 respectively, and the mean fluorescence intensity (MFI) of different clones binding to antigen was compared. The higher the MFI, the stronger the affinity with antigen. According to the intensity of fluorescence signal, 17 clones of YC208B3, YC208E2, YC208G3, YC210H2, YC215D1, YC215F3, YC215H3, YC215H5, YC216D5, YC216F1, YC216F2, YC208E1, YC209C5, YC209D4, YC209D6, YC209E6, and YC214E2 etc. were screened out for further determination of the binding curve Ag₅₀ to human and monkey PD-1.

The clones were distributed in columns, and placed on a 96-well plate with 1.0 × 10⁵ cells/well. After washing and centrifugation with 1 × PBSA, gradient diluted human and monkey PD-1 antigens were added and incubated at room temperature for 30 minutes; after a centrifugation the supernatant was removed, 100 µL of fluorescent secondary antibody was added, and incubated for 30 minutes in the dark; after washing, it was resuspended in 30 µL 1 x PBSA, and corresponding fluorescence signals were read by flow cytometry. The data were fitted by GraphPad Prism 8 software, and the binding curve Ag₅₀ of each clone to human and monkey PD-1 was calculated. Results are shown in Table 4.

**Table 4. Assay of Binding of Mutant Clones to Human and Monkey PD-1**

| Clone Number | Human PD-1 Ag₅₀ (nM) | Monkey PD-1 Ag₅₀ (nM) |
|---|---|---|
| 41D2HzL4H3 | 5.08 | 36.10 |
| YC208B3 | 1.07 | 3.41 |
| YC208E1 | 0.72 | 52.34 |
| YC208E2 | 0.58 | 3.28 |
| YC208G3 | 1.50 | 11.03 |
| YC209C5 | 1.29 | 5.88 |
| YC209D4 | 1.36 | 15.76 |
| YC209D6 | 0.43 | 31.41 |
| YC209E6 | 1.90 | 14.32 |
| YC210H2 | 1.36 | 7.16 |
| YC214E2 | 1.04 | 24.10 |
| YC215D1 | 1.70 | 4.16 |
| YC215F3 | 5.49 | 13.75 |
| YC215H3 | 1.26 | 2.42 |
| YC215H5 | 1.28 | 1.89 |
| YC216D5 | 1.92 | 3.41 |
| YC216F1 | 1.32 | 7.59 |
| YC216F2 | 1.78 | 12.72 |

Based on the sequence of each clone as well as the Ag₅₀ values for human and monkey PD-1, or the like, mutant sequences of 10 clones such as YC208B3, YC208E2, YC208G3, YC209C5, YC209D6, YC215D1, YC215F3, YC215H3, YC216D5, YC216F2 etc. were selected, the expression plasmid was constructed with IgG1 LALA D265S subclass, and the antibodies were named as Ab2005Am01-10.

### 7.2 Expression of Candidate Antibodies

The heavy chain variable region sequence of each clone was fused with IgG1 LALA D265S, and the light chain variable region was fused with Kappa chain constant region. The gene was synthesized and introduced into an expression vector pcDNA3.4(Life Technologies). The light and heavy expression plasmids were transferred into ExpiCHO cells (ThermoFisher Scientific, A29133), and the antibody was transiently expressed according to the manufacturer's method of the ExpiCHO expression system, and the process was as follows: Expi CHO cells were cultured to a density of 6×10⁶/mL in a culture medium with a total culture volume of 25 mL at 36.5°C and 8% carbon dioxide concentration, and 10 µg of antibody light expression plasmids and heavy chain expression plasmids respectively were transferred to the cells with an ExpiFectamine transfection reagent. One day after transfection, 150 µL and 4 mL ExpiCHO Enhancer and ExpiCHO Supplement respectively were added into the cultured cells, the cells were continued to be cultured to day 9, then the supernatant was taken following centrifugation at 4 degrees and 3500 rpm. AmMagTM Protein A magnetic beads (Genscript, L00695) and antibody expression supernatant were mixed, incubated at room temperature for 2 hours, and then the supernatant was taken following being washed twice with PBS, and an appropriate amount of elution buffer Protein G or A SefinoseTM Elution buffer (Sangon, C600481) was added, mixed uniformly, and placed on a tube rack for static incubation for 5 minutes. During the incubation, the magnetic beads was resuspended for 2 to 3 times, and the elution repeated for 2 times. After elution, it was neutralized by immediately adding an appropriate amount of neutralizing solution 1M Tris-HCl, pH7.5(Sangon, B548124) and be ready to use, and thereby obtaining purified antibodies as see in Table 5.

**Table 5. Data for Expression and Purification of Candidate Antibodies**

| Antibody Number | Clone Number | Isoelectric Point | Extinction Coefficient | Batch Number | Expression Amount (mg/L) |
|---|---|---|---|---|---|
| Ab2005Am01 | YC208B3 | 8.1 | 1.53 | 20210621A096 | 73.7 |
| Ab2005Am02 | YC208E2 | 8.1 | 1.59 | 20210622A051 | 72.7 |
| Ab2005Am03 | YC208G3 | 8.1 | 1.51 | 20210621A097 | 74.4 |
| Ab2005Am04 | YC209C5 | 8.1 | 1.53 | 20210621A098 | 70.7 |
| Ab2005Am05 | YC209D6 | 8.2 | 1.51 | 20210621A099 | 64.4 |
| Ab2005Am06 | YC215D1 | 8.2 | 1.57 | 20210621A100 | 68.7 |
| Ab2005Am07 | YC215F3 | 8.1 | 1.49 | 20210621A101 | 70.7 |
| Ab2005Am08 | YC215H3 | 8.2 | 1.51 | 20210622A052 | 70.7 |
| Ab2005Am09 | YC216D5 | 8.1 | 1.49 | 20210621A102 | 64.7 |
| Ab2005Am 10 | YC216F2 | 8.1 | 1.49 | 20210621A103 | 77.7 |

### 7.3 Evaluation of Physical and Chemical Properties of Candidate Antibodies

The antibodies Ab2005Am01-10 were used as candidate molecules to continue the evaluation for physical and chemical druggability, and the details were as follows:

### SEC-HPLC Purity Analysis

(1) The concentration of sample was adjusted to 1 mg/mL, mixed uniformly, centrifuged at 12000 rpm for 5 min, the supernatant was obtained and transferred to a sample vial, and put into an HPLC sample tray. Chromatographic conditions were as follows:

| Chromatographic Condition | Parameter |
|---|---|
| Chromatographic Column | TSK G3000SWxl |
| Detection Wavelength | 280 nm |
| Column Temperature | 25 °C |
| Sample Chamber Temperature | 5 °C |
| Flow Rate | 0.5 mL/min |

(2) After the chromatographic column was balanced with mobile phase (200 mM phosphate buffer solution, pH6.8), the sample was injected and analyzed, and the data was analyzed by chromatographic software, the peak area percentage of each peak was calculated by peak area normalization method. The higher the percentage, the higher the purity of the antibody.

### HIC-HPLC Analysis

(1) The concentration of sample was adjusted to 1 mg/ml, after a centrifugation the supernatant was taken to be detected. Chromatographic conditions were as follows:

| Chromatographic Condition | Parameter | |
|---|---|---|
| Chromatographic Column | MAbPac^{™}HIC-10 | |
| Detection Wavelength | | 214 nm |
| Column Temperature | | 30 °C |
| Sample Chamber Temperature | | 5 °C |
| Flow Rate | | 0.8 mL/min |

(2) Gradient elution was carried out with mobile phase A (50 mM phosphate buffer/1 M ammonium sulfate, pH 7.0) and mobile phase B (50 mM phosphate buffer, pH 7.0), and the retention time of the major peak was recorded. A short peak time shows a strong hydrophilicity of the antibody.

### Analysis of Melting Temperature (Tm) Value

The concentration of the sample was adjusted to 1 mg/mL, then 13 µL of the solution of test sample was taken and added into the PCR tube according to the instructions of Protein Thermal Shift^{™} Starter Kit, 5 µL Protein Thermal Shift^{™} Buffer was added, and 2 µL 10× dye solution was added to make a reaction volume of 20 µL, mixed uniformly, and the mixture was centrifuged at 12000 rpm for 5 min to remove bubbles. The test sample was placed in a PCR instrument for sample analysis, and Tm value of the sample was recorded. The higher the Tm value, the better the thermal stability of the antibody.

### iCIEF Analysis

The sample solution was taken and added into the following systems that had been mixed uniformly: 1% methyl cellulose (MC) of 70 µL, urea 5 M of 80 µL, ampholyte Pharmalyte pH 3-10 of 8 µL, pI marker 5.5 and 9.5 of 2 µL respectively. An appropriate volume of ultrapure water was added up to 200 µL and mixed uniformly. The mixture was centrifuged and the supernatant was taken and analyzed. After the analyzation, the result file was imported into ChromPerfect software for spectrum integration and the isoelectric point and percentage of each peak were calculated.

The physical and chemical properties of the candidate antibodies Ab2005Am01-10 were summarized in Table 6. The results show that the purity, thermal stability, hydrophilicity and other physical and chemical indexes of the candidate antibodies were excellent, and the *in vitro* activity of the candidate antibodies were subsequently evaluated.

**Table 6. Analysis Results of physical and chemical properties of candidate antibodies**

| Antibody Number | Purity | Tm (°C) | pI | HIC (min) | Acid Peak % | Major Peak % | Alkali Peak % |
|---|---|---|---|---|---|---|---|
| Ab2005Am01 | 98.66% | 83.3 | 9.0 | 16.3 | 25.5 | 72.4 | 2.1 |
| Ab2005Am02 | 98.83% | 84.1 | 9.0 | 17.8 | 22.5 | 74.4 | 3.2 |
| Ab2005Am03 | 98.80% | 83.0 | 9.0 | 15.3 | 24.5 | 73.6 | 1.9 |
| Ab2005Am04 | 98.92% | 82.6 | 9.0 | 20.6 | 24.3 | 73.7 | 2.0 |
| Ab2005Am05 | 99.00% | 83.3 | 9.2 | 13.8 | 24.1 | 73.7 | 2.2 |
| Ab2005Am06 | 99.33% | 83.6 | 9.2 | 13.9 | 25.4 | 70.8 | 3.8 |
| Ab2005Am07 | 98.82% | 85.3 | 9.0 | 13.3 | 22.2 | 74.7 | 3.1 |
| Ab2005Am08 | 98.64% | 85.0 | 9.1 | 13.4 | 19.6 | 75.1 | 5.3 |
| Ab2005Am09 | 98.78% | 84.1 | 9.0 | 14.9 | 24.1 | 73.3 | 2.6 |
| Ab2005Am10 | 98.79% | 85.3 | 9.0 | 15.1 | 22.5 | 74.5 | 3.0 |

### 7.4 Affinity Assay of Candidate Antibodies

In order to determine the affinity of candidate antibodies Ab2005Am01-10 to human or monkey PD-1, the affinity of candidate antibodies to human PD-1(Acro, Catalog Number: PD-1-H5221) or monkey PD-1(Acro, Catalog Number: PD-1-C5223) was determined by using Octet RED96e (Fortebio); both the antigens and the antibodies were diluted with 1×PBST (1×PBS: Sangon, B548117-0500; 0.02% Tween 20: Sigma, P1379), the concentration of antigen for use was 100 nM, and the concentration of antibody for use was 50 nM. The sample of the candidate antibody was added into a 96-well plate (Greiner bio-one, 655209) by 200 µL/well, the software parameters were set with a temperature of 30°C and a frequency of 5.0 Hz for collecting the standard kinetic signals; AHC sensor (Fortébio, Catalog Number: 18-0015) was prewet with 1×PBST for 10 minutes, and then detection was carried on with the instrument. Each cycle comprised the following steps: 1) immersing in buffer for 60 s; 2) detecting whether the antigen has nonspecific binding with the sensor; 3) regenerating 10 mm glycine solution with pH 1.7; 4) immersing in buffer for 60 s; 5) immobilizing the antibody on the sensor for 13 s; 6) immersing the sensor in buffer for 180 s; 7) the antigen binding to the antibody for 180 s; 8) dissociating the antigen from the antibody for 10 minutes; 9) regenerating the sensor. The association rate (Kₒₙ) and dissociation rate (K_{off}) of the antigen-antibody at a ratio of 1:1 were determined by Fortebio's Data Analysis 12.0 software, and thereby the equilibrium dissociation constant (*K_{D}*) of antibodies were calculated. The results are shown in Tables 7 and 8.

**Table 7. Affinity Assay of Candidate Antibodies to Human PD-1**

| Antibody Number | Response Value | *K_{D}* (M) | kon(1/Ms) | kdis(1/s) |
|---|---|---|---|---|
| 41D2HzL4H3 | 0.15 | 1.27E-09 | 4.64E+05 | 5.89E-04 |
| Ab2005Am01 | 0.13 | 7.11E-10 | 4.87E+05 | 3.46E-04 |
| Ab2005Am02 | 0.15 | 1.10E-09 | 4.27E+05 | 4.70E-04 |
| Ab2005Am03 | 0.16 | 9.07E-10 | 4.02E+05 | 3.65E-04 |
| Ab2005Am04 | 0.17 | 8.36E-10 | 4.47E+05 | 3.74E-04 |
| Ab2005Am05 | 0.16 | 5.29E-10 | 4.93E+05 | 2.61E-04 |
| Ab2005Am06 | 0.15 | 7.79E-10 | 4.17E+05 | 3.25E-04 |
| Ab2005Am07 | 0.15 | 1.31E-09 | 4.93E+05 | 6.46E-04 |
| Ab2005Am08 | 0.17 | 5.04E-10 | 4.57E+05 | 2.30E-04 |
| Ab2005Am09 | 0.15 | 4.63E-10 | 4.79E+05 | 2.22E-04 |
| Ab2005Am 10 | 0.15 | 6.48E-10 | 4.86E+05 | 3.15E-04 |

**Table 8. Affinity Assay of Candidate Antibodies to Monkey PD-1**

| Antibody Number | Response Value | *K_{D}* (M) | kon(1/Ms) | kdis(1/s) |
|---|---|---|---|---|
| 41D2HzL4H3 | 0.14 | 5.20E-09 | 2.95E+05 | 1.53E-03 |
| Ab2005Am01 | 0.14 | 2.19E-09 | 3.04E+05 | 6.63E-04 |
| Ab2005Am02 | 0.15 | 4.14E-09 | 2.65E+05 | 1.10E-03 |
| Ab2005Am03 | 0.17 | 4.24E-09 | 2.77E+05 | 1.18E-03 |
| Ab2005Am04 | 0.17 | 7.54E-09 | 3.17E+05 | 2.39E-03 |
| Ab2005Am05 | 0.13 | 1.81E-08 | 2.55E+05 | 4.62E-03 |
| Ab2005Am06 | 0.14 | 2.80E-09 | 2.34E+05 | 6.55E-04 |
| Ab2005Am07 | 0.15 | 3.62E-09 | 3.10E+05 | 1.12E-03 |
| Ab2005Am08 | 0.17 | 1.52E-09 | 3.20E+05 | 4.86E-04 |
| Ab2005Am09 | 0.16 | 2.06E-09 | 3.82E+05 | 7.88E-04 |
| Ab2005Am10 | 0.16 | 2.63E-09 | 3.48E+05 | 9.16E-04 |

The results showed that the affinities of candidate antibodies Ab2005Am06, 08, 09 to human and monkey PD-1 were about 2 times greater than the parent antibodies before affinity maturation, and the evaluations for *in vitro* binding and functional experiment were subsequently performed.

### 7.5 Binding of Candidate Antibodies to Human and monkey PD-1 on Cell Surfaces

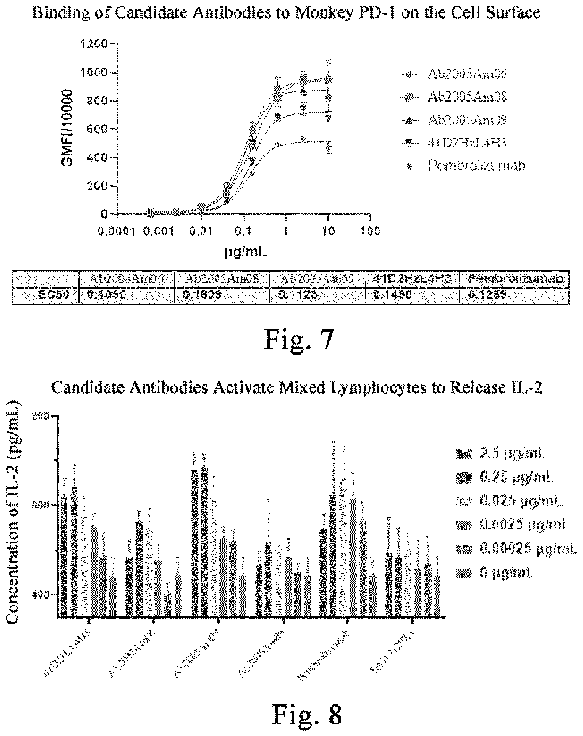

In order to determine the binding of candidate antibodies Ab2005Am06, 08 and 09 to human and monkey PD-1 on the cell surface, CHOK-hPD-1 (GenScript, M00529) cells or CHOK-cynoPD-1 (GenScript, M00572) were collected and washed with FACS buffer (containing 2% FBS in PBS) followed by resuspension, and divide into a 96-well plate by 1E5/50 µL/well; candidate antibodies Ab2005Am06, 08 and 09 were diluted from the initial concentration of 10 µg/mL with a four-fold gradient; the diluted antibody solution was added into the above 96-well U-shaped plate, 50 µL/well, mixed uniformly and incubated at 4°C for 1 hour; then it was centrifuged at 2000 rpm for 5 minutes and the supernatant was removed; 200 µL of FACS buffer was added into each well to wash once, Alexa Fluor 488 secondary antibody (Invitrogen, A-11013) diluted at 1:1000 was added, 100 µL/well, mixed uniformly and incubated at 4°C for 1 hour; then it was centrifuged at 2000 rpm for 5 minutes and the supernatant was removed; 200 µL of FACS buffer was added into each well to wash once, and it was resuspended with 30 µL FACS buffer for detection on the machine. The data were processed by GraphPad Prism software, and the experimental results showed that candidate antibodies could bind to human and monkey PD-1 on the cell surface. Fig. 6 shows the binding results of candidate antibodies to human PD-1 on the cell surface. Fig. 7 shows the binding results of candidate antibodies to monkey PD-1 on the cell surface.

### 7.6 Functional Experiment of Candidate Antibodies Activating Mixed Lymphocytes to Release IL-2

In order to compare the *in vitro* activities of candidate antibodies Ab2005Am06, 08, 09 with the parent antibody 41D2HzL4H3 and the benchmark antibody Pembrolizumab, it was carried out in the same experiment of activating mixed lymphocytes to release IL-2 as follows: human dendritic DC cells were resuscitated with a cell culture medium (1640 + 2% FBS), the density of DC cells was adjusted to 1×10⁵ to 1×10⁷ cells/mL, then mitomycin C with a final concentration of 50 µg/mL was added, the mixture was treated at 37 degrees in the dark for 30 minutes, then 10 mL culture medium was added for termination, it was centrifuged at 400 g for 10 minutes, and then washed with 10 mL culture medium. Gradient dilution of anti-PD1 antibody: the greatest final concentration of antibody was 2.5 µg/mL (preparation concentration was 10 µg/mL), and the gradient dilution is performed by ten-fold gradient dilution (5 concentration points + one 0 concentration point), and then 50 µL of prepared anti-PD1 antibody was added into the corresponding cell culture plate (Corning, Catalog Number: 3599). DC cells treated with human peripheral blood lymphocytes PBMC and mitomycin C were collected, the density of DC cells was adjusted to 2×10⁵ cells/mL, and then the cells added into the culture plate, with 50 µL/well, i.e., the number of DC cells is 1×10⁴ cells/well; the density of PBMC cells was adjusted to 2×10⁶ cells/mL, and then the cells were added into the culture plate, with 100 µL/well, i.e., the number of PBMC cells was 2×10⁵ cells/well. The cell culture plate was placed in a cell incubator with 5% carbon dioxide and incubated at 37°C for 3 days. After 3 days, the mixture was centrifuged with 300g for 5 minutes. The supernatant was collected and content of IL-2 was detected with Human IL-2 ELISA kit (Novus, Catalog Number: VAL110), the detection method was strictly performed according to the kit instructions. The data were processed by GraphPad Prism software, and the results showed that candidate antibodies were able to activate mixed lymphocytes to release IL-2. Fig. 8 shows the results that candidate antibodies activated mixed lymphocytes to release IL-2.

In summary, after affinity maturation, the candidate antibody Ab2005Am08 (HCDR2 is SGGGRY, as set forth in SEQ ID NO: 85; VH is EVQLVESGGGLVKPGGSLRLSCAASGFTFSNYDMSWVRQAPGKGLEWVSTISGGGR YTYYADSVKGRFTISRDNAKNSLYLQMNSLRAEDTAVYYCVSPYYGMEYWGQGTLV TVSS, as set forth in SEQ ID NO: 86) had greater binding ability to human and monkey PD-1 compared with the parent antibody 41D2HzL4H3, and it performs better than the parent antibody and the benchmark antibody Pembrolizumab in the experiment of activating mixed lymphocytes to release IL-2 *in vitro*; meanwhile, the candidate antibody Ab2005Am08 was compliant with druggability standards in terms of expression amount as well as physical and chemical properties, etc., thereby having the potential for further development.

## Claims

1. An isolated antigen-binding protein having one or more of the following properties:
(1) capable of binding to PD-1 protein derived from a primate with a *K_{D}* value of 1×10⁸ M or less;
(2) capable of blocking the binding of PD-1 protein to PD-L1 protein;
(3) capable of blocking the binding of PD-1 protein to PD-L2 protein; and
(4) capable of stimulating the secretion of cytokines in immune cells.

2. The isolated antigen-binding protein according to claim 1, wherein the primate includes a human and/or a monkey.

3. The isolated antigen-binding protein according to claim 1 or 2, including an antibody or an antigen-binding fragment thereof.

4. The isolated antigen-binding protein according to claim 3, wherein the antigen-binding fragment includes a Fab, a Fab', a F(ab)₂, a Fv fragment, a F(ab')₂, a scFv, a di-scFv, a VHH and/or a dAb.

5. The isolated antigen-binding protein according to any one of claims 3 to 4, wherein the antibody is selected from the group consisting of a monoclonal antibody, a chimeric antibody, a humanized antibody and a fully human antibody.

6. The isolated antigen-binding protein according to any one of claims 1 to 5, wherein the isolated antigen-binding protein is able to compete with a reference antibody for binding to PD-1 protein, wherein the reference antibody comprises a heavy chain variable region VH and a light chain variable region VL, the VH of the reference antibody comprises HCDR1, HCDR2 and HCDR3, the VL of the reference antibody comprises LCDR1, LCDR2 and LCDR3, and the reference antibody comprises any one of groups of amino acid sequences selected from:
(1) HCDR1: SEQ ID NO: 1, HCDR2: SEQ ID NO: 2, HCDR3: SEQ ID NO: 3, LCDR1: SEQ ID NO: 8, LCDR2: SEQ ID NO: 9, and LCDR3: SEQ ID NO: 10;
(2) HCDR1: SEQ ID NO: 27, HCDR2: SEQ ID NO: 28, HCDR3: SEQ ID NO: 29, LCDR1: SEQ ID NO: 34, LCDR2: SEQ ID NO: 35, and LCDR3: SEQ ID NO: 36; and
(3) HCDR1: SEQ ID NO: 1, HCDR2: SEQ ID NO: 85, HCDR3: SEQ ID NO: 3, LCDR1: SEQ ID NO: 8, LCDR2: SEQ ID NO: 9, and LCDR3: SEQ ID NO: 10.

7. The according to any one of claims 1 to 6, wherein the isolated antigen-binding protein comprises HCDR3, and the HCDR3 comprises an amino acid sequence as set forth in SEQ ID NO: 3 or SEQ ID NO: 29.

8. The isolated antigen-binding protein according to any one of claims 1 to 7, wherein the isolated antigen-binding protein comprises HCDR2, and the HCDR2 comprises an amino acid sequence as set forth in SEQ ID NO: 2, SEQ ID NO: 28 or SEQ ID NO: 85.

9. The isolated antigen-binding protein according to any one of claims 1 to 8, wherein the isolated antigen-binding protein comprises HCDR1, and the HCDR1 comprises an amino acid sequence as set forth in SEQ ID NO: 1 or SEQ ID NO: 27.

10. The isolated antigen-binding protein according to any one of claims 1 to 9, comprising HCDR1, HCDR2 and HCDR3, wherein the HCDR1 comprises an amino acid sequence as set forth in any one of SEQ ID NO: 1 and SEQ ID NO: 27, the HCDR2 comprises an amino acid sequence as set forth in any one of SEQ ID NO: 2, SEQ ID NO: 28 and SEQ ID NO: 85, and the HCDR3 comprises an amino acid sequence as set forth in any one of SEQ ID NO: 3 and SEQ ID NO: 29.

11. The isolated antigen-binding protein according to any one of claims 1 to 10, wherein the isolated antigen-binding protein comprises HCDR1, HCDR2 and HCDR3, and the HCDR1, HCDR2 and HCDR3 are selected from any one of groups of amino acid sequences as follows:
(1) HCDR1: SEQ ID NO: 1, HCDR2: SEQ ID NO: 2 and HCDR3: SEQ ID NO: 3;
(2) HCDR1: SEQ ID NO: 27, HCDR2: SEQ ID NO: 28 and HCDR3: SEQ ID NO: 29; and
(3) HCDR1: SEQ ID NO: 1, HCDR2: SEQ ID NO: 85 and HCDR3: SEQ ID NO: 3.

12. The isolated antigen-binding protein according to any one of claims 1 to 11, comprising a heavy chain variable region VH, wherein the VH comprises a framework region H-FR1, and the C-terminus of the H-FR1 is directly or indirectly linked to the N-terminus of the HCDR1, and the H-FR1 comprises an amino acid sequence as set forth in SEQ ID NO: 57 or SEQ ID NO: 58.

13. The isolated antigen-binding protein according to claim 12, wherein the H-FR1 comprises an amino acid sequence as set forth in any one of SEQ ID NO: 4, SEQ ID NO: 17, SEQ ID NO: 30 and SEQ ID NO: 43.

14. The isolated antigen-binding protein according to any one of claims 12 to 13, wherein the VH comprises a framework region H-FR2, the H-FR2 is located between the HCDR1 and the HCDR2, and the H-FR2 comprises an amino acid sequence as set forth in SEQ ID NO: 59 or SEQ ID NO: 60.

15. The isolated antigen-binding protein according to claim 14, wherein the H-FR2 comprises an amino acid sequence as set forth in any one of SEQ ID NO: 5, SEQ ID NO: 18, SEQ ID NO: 31 and SEQ ID NO: 44.

16. The isolated antigen-binding protein according to any one of claims 12 to 15, wherein the VH comprises a framework region H-FR3, the H-FR3 is located between the HCDR2 and the HCDR3, and the H-FR3 comprises an amino acid sequence as set forth in SEQ ID NO: 61 or SEQ ID NO: 62.

17. The isolated antigen-binding protein according to claim 16, wherein the H-FR3 comprises an amino acid sequence as set forth in any one of SEQ ID NO: 6, SEQ ID NO: 19, SEQ ID NO: 32 and SEQ ID NO: 45.

18. The isolated antigen-binding protein according to any one of claims 12 to 17, wherein the VH comprises a framework region H-FR4, the N-terminus of the H-FR4 is linked to the C-terminus of the HCDR3, and the H-FR4 comprises an amino acid sequence as set forth in SEQ ID NO: 63 or SEQ ID NO: 64.

19. The isolated antigen-binding protein according to claim 18, wherein the H-FR4 comprises an amino acid sequence as set forth in any one of SEQ ID NO: 7, SEQ ID NO: 20, SEQ ID NO: 33 and SEQ ID NO: 46.

20. The isolated antigen-binding protein according to any one of claims 1 to 19, comprising H-FR1, H-FR2, H-FR3 and H-FR4, wherein the H-FR1 comprises an amino acid sequence as set forth in any one of SEQ ID NO: 4, SEQ ID NO: 17, SEQ ID NO: 30 and SEQ ID NO: 43, the H-FR2 comprises an amino acid sequence as set forth in any one of SEQ ID NO: 5, SEQ ID NO: 18, SEQ ID NO: 31 and SEQ ID NO: 44, the H-FR3 comprises an amino acid sequence as set forth in any one of SEQ ID NO: 6, SEQ ID NO: 19, SEQ ID NO: 32 and SEQ ID NO: 45 and the H-FR4 comprises an amino acid sequence as set forth in any one of SEQ ID NO: 7, SEQ ID NO: 20, SEQ ID NO: 33 and SEQ ID NO: 46.

21. The isolated antigen-binding protein according to any one of claims 1 to 20, wherein the isolated antigen-binding protein comprises H-FR1, H-FR2, H-FR3 and H-FR4, and the H-FR1, H-FR2, H-FR3 and H-FR4 are selected from any one of groups of amino acid sequences as follows:
(1) H-FR1: SEQ ID NO: 4, H-FR2: SEQ ID NO: 5, H-FR3: SEQ ID NO: 6 and H-FR4: SEQ ID NO: 7;
(2) H-FR1: SEQ ID NO: 17, H-FR2: SEQ ID NO: 18, H-FR3: SEQ ID NO: 19 and H-FR4: SEQ ID NO: 20;
(3) H-FR1: SEQ ID NO: 30, H-FR2: SEQ ID NO: 31, H-FR3: SEQ ID NO: 32 and H-FR4: SEQ ID NO: 33; and
(4) H-FR1: SEQ ID NO: 43, H-FR2: SEQ ID NO: 44, H-FR3: SEQ ID NO: 45 and H-FR4: SEQ ID NO: 46.

22. The isolated antigen-binding protein according to any one of claims 1 to 21, wherein the isolated antigen-binding protein comprises a heavy chain variable region VH, and the VH comprises an amino acid sequence as set forth in SEQ ID NO: 65 or SEQ ID NO: 66.

23. The isolated antigen-binding protein according to claim 22, wherein the VH comprises an amino acid sequence as set forth in any one of SEQ ID NO: 15, SEQ ID NO: 25, SEQ ID NO: 41, SEQ ID NO: 51 and SEQ ID NO: 86.

24. The isolated antigen-binding protein according to any one of claims 1 to 23, wherein the isolated antigen-binding protein comprises a heavy chain constant region, and the heavy chain constant region is derived from a human IgG constant region.

25. The isolated antigen-binding protein according to claim 24, wherein the heavy chain constant region is derived from a human IgG4 constant region, and the human IgG4 constant region comprises an amino acid sequence as set forth in SEQ ID NO: 77 or 81.

26. The isolated antigen-binding protein according to any one of claims 1 to 22, wherein the isolated antigen-binding protein comprises an antibody heavy chain HC, and the HC comprises an amino acid sequence as set forth in SEQ ID NO:79, 83 or 87.

27. The isolated antigen-binding protein according to any one of claims 1 to 26, wherein the isolated antigen-binding protein comprises LCDR3, and the LCDR3 comprises an amino acid sequence as set forth in SEQ ID NO: 10 or SEQ ID NO: 36.

28. The isolated antigen-binding protein according to any one of claims 1 to 27, wherein the isolated antigen-binding protein comprises LCDR2, and the LCDR2 comprises an amino acid sequence as set forth in SEQ ID NO: 9 or SEQ ID NO: 35.

29. The isolated antigen-binding protein according to any one of claims 1 to 28, wherein the isolated antigen-binding protein comprises LCDR1, and the LCDR1 comprises an amino acid sequence as set forth in SEQ ID NO: 8 or SEQ ID NO: 34.

30. The isolated antigen-binding protein according to any one of claims 1 to 29, comprising LCDR1, LCDR2 and LCDR3, wherein the LCDR1 comprises an amino acid sequence as set forth in any one of SEQ ID NO: 8 and SEQ ID NO: 34, the LCDR2 comprises an amino acid sequence as set forth in any one of SEQ ID NO: 9 and SEQ ID NO: 35, and the LCDR3 comprises an amino acid sequence as set forth in any one of SEQ ID NO: 10 and SEQ ID NO: 36.

31. The isolated antigen-binding protein according to any one of claims 1 to 30, wherein the isolated antigen-binding protein comprises LCDR1, LCDR2 and LCDR3, and the LCDR1, LCDR2 and LCDR3 are selected from any one of groups of amino acid sequences as follows:
(1) LCDR1: SEQ ID NO: 34, LCDR2: SEQ ID NO: 35 and LCDR3: SEQ ID NO: 36; and
(2) LCDR1: SEQ ID NO: 8, LCDR2: SEQ ID NO: 9 and LCDR3: SEQ ID NO: 10.

32. The isolated antigen-binding protein according to any one of claims 1 to 31, wherein the isolated antigen-binding protein comprises a light chain variable region VL, wherein the VL comprises a framework region L-FR1, the C-terminus of the L-FR1 is directly or indirectly linked to the N-terminus of the LCDR1, and the L-FR1 comprises an amino acid sequence as set forth in SEQ ID NO: 69 or SEQ ID NO: 70.

33. The isolated antigen-binding protein according to claim 32, wherein the L-FR1 comprises an amino acid sequence as set forth in any one of SEQ ID NO: 11, SEQ ID NO: 21, SEQ ID NO: 37 and SEQ ID NO: 47.

34. The isolated antigen-binding protein according to any one of claims 32-33, wherein the VL comprises a framework region L-FR2, the L-FR2 is located between the LCDR1 and the LCDR2, and the L-FR2 comprises an amino acid sequence as set forth in SEQ ID NO: 71 or SEQ ID NO: 72.

35. The isolated antigen-binding protein according to claim 34, wherein the L-FR2 comprises an amino acid sequence as set forth in any one of SEQ ID NO: 12, SEQ ID NO: 22, SEQ ID NO: 38 and SEQ ID NO: 48.

36. The isolated antigen-binding protein according to any one of claims 32 to 35, wherein the VL comprises a framework region L-FR3, the L-FR3 is located between the LCDR2 and the LCDR3, and the L-FR3 comprises an amino acid sequence as set forth in SEQ ID NO: 73 or SEQ ID NO: 74.

37. The isolated antigen-binding protein according to claim 36, wherein the L-FR3 comprises an amino acid sequence as set forth in any one of SEQ ID NO: 13, SEQ ID NO: 23, SEQ ID NO: 39 and SEQ ID NO: 49.

38. The isolated antigen-binding protein according to any one of claims 32 to 37, wherein the N-terminus of the L-FR4 is linked to the C-terminus of the LCDR3, and the L-FR4 comprises an amino acid sequence as set forth in SEQ ID NO: 75 or SEQ ID NO: 76.

39. The isolated antigen-binding protein according to claim 38, wherein the L-FR4 comprises an amino acid sequence as set forth in any one of SEQ ID NO: 14, SEQ ID NO: 24, SEQ ID NO: 40 and SEQ ID NO: 50.

40. The isolated antigen-binding protein according to any one of claims 1 to 39, comprising L-FR1, L-FR2, L-FR3 and L-FR4, wherein the L-FR1 comprises an amino acid sequence as set forth in any one of SEQ ID NO: 11, SEQ ID NO: 21, SEQ ID NO: 37 and SEQ ID NO: 47, the L-FR2 comprises an amino acid sequence as set forth in any one of SEQ ID NO: 12, SEQ ID NO: 22, SEQ ID NO: 38 and SEQ ID NO: 48, the L-FR3 comprises an amino acid sequence as set forth in any one of SEQ ID NO: 13, SEQ ID NO: 23, SEQ ID NO: 39 and SEQ ID NO: 49, and the L-FR4 comprises an amino acid sequence as set forth in any one of SEQ ID NO: 14, SEQ ID NO: 24, SEQ ID NO: 40 and SEQ ID NO: 50.

41. The isolated antigen-binding protein according to any one of claims 1 to 40, wherein the isolated antigen-binding protein comprises L-FR1, L-FR2, L-FR3 and L-FR4, and the L-FR1, L-FR2, L-FR3 and L-FR4 are selected from any one of groups of amino acid sequences as follows:
(1) L-FR1: SEQ ID NO: 11, L-FR2: SEQ ID NO: 12, L-FR3: SEQ ID NO: 13 and L-FR4: SEQ ID NO: 14;
(2) L-FR1: SEQ ID NO: 21, L-FR2: SEQ ID NO: 22, L-FR3: SEQ ID NO: 23 and L-FR4: SEQ ID NO: 24;
(3) L-FR1: SEQ ID NO: 37, L-FR2: SEQ ID NO: 38, L-FR3: SEQ ID NO: 39 and L-FR4: SEQ ID NO: 40; and
(4) L-FR1: SEQ ID NO: 47, L-FR2: SEQ ID NO: 48, L-FR3: SEQ ID NO: 49 and L-FR4: SEQ ID NO: 50.

42. The isolated antigen-binding protein according to any one of claims 1 to 41, wherein the isolated antigen-binding protein comprises a light chain variable region VL, and the VL comprises an amino acid sequence as set forth in SEQ ID NO: 67 or SEQ ID NO: 68.

43. The isolated antigen-binding protein according to any one of claims 1 to 42, wherein the VL comprises an amino acid sequence as set forth in any one of SEQ ID NO: 16, SEQ ID NO: 26, SEQ ID NO: 42 and SEQ ID NO: 52.

44. The isolated antigen-binding protein according to any one of claims 1 to 43, wherein the isolated antigen-binding protein comprises an antibody light chain constant region, and the antibody light chain constant region comprises an amino acid sequence as set forth in SEQ ID NO: 78 or 82.

45. The isolated antigen-binding protein according to any one of claims 1 to 38, wherein the isolated antigen-binding protein comprises an antibody light chain LC, and the LC comprises an amino acid sequence as set forth in SEQ ID NO: 80 or 84.

46. A polypeptide comprising the isolated antigen-binding protein of any one of claims 1 to 45.

47. An immunoconjugate comprising the isolated antigen-binding protein of any one of claims 1 to 45 or the polypeptide of claim 46.

48. An isolated nucleic acid molecule(s) encoding the isolated antigen-binding protein of any one of claims 1 to 45.

49. A vector comprising the nucleic acid molecule according to claim 48.

50. A cell comprising the nucleic acid molecule according to claim 48 or the vector according to claim 49.

51. A method for preparing the isolated antigen-binding protein of any one of claims 1 to 45, comprising culturing the cell according to claim 50 under conditions that the isolated antigen-binding protein of any one of claims 1 to 45 can be expressed.

52. A pharmaceutical composition comprising the isolated antigen-binding protein of any one of claims 1 to 45, the polypeptide of claim 46, the immunoconjugate of claim 47, the nucleic acid molecule of claim 48, the vector of claim 49 and/or the cell of claim 50; and optionally a pharmaceutically acceptable carrier.

53. Use of the isolated antigen-binding protein of any one of claims 1 to 45, the polypeptide of claim 46, the immunoconjugate of claim 47, the nucleic acid molecule of claim 48, the vector of claim 49, the cell of claim 50 and/or the pharmaceutical composition of claim 52 in the manufacture of a medicament for preventing, alleviating and/or treating a tumor.

54. The use according to claim 53, wherein the tumor includes a tumor with a high expression of PD-1 or PD-L1.

55. The use according to any one of claims 53 to 54, wherein the tumor includes a solid tumor and/or a non-solid tumor.

56. The use according to any one of claims 53 to 55, wherein the tumor includes melanoma, lung cancer, renal cancer, esophageal cancer, head and neck cancer, lymphoma, liver cancer and/or gastric cancer.

57. A method for preventing, alleviating or treating a tumor, comprising administering the isolated antigen-binding protein of any one of claims 1 to 45, the polypeptide of claim 46, the immunoconjugate of claim 47, the nucleic acid molecule of claim 48, the vector of claim 49, the cell of claim 50 and/or the pharmaceutical composition of claim 52 to a subject in need.

58. The method according to claim 57, wherein the tumor includes a tumor with a high expression of PD-1 or PD-L1.

59. The method according to any one of claims 57 to 58, wherein the tumor includes a solid tumor and/or a non-solid tumor.

60. The method according to any one of claims 57 to 59, wherein the tumor includes melanoma, lung cancer, renal cancer, esophageal cancer, head and neck cancer, lymphoma, liver cancer and/or gastric cancer.

61. The isolated antigen-binding protein of any one of claims 1 to 45, the polypeptide of claim 46, the immunoconjugate of claim 47, the nucleic acid molecule of claim 48, the vector of claim 49, the cell of claim 50 and/or the pharmaceutical composition of claim 52, for use in preventing, alleviating or treating a tumor.

62. The isolated antigen-binding protein, the polypeptide, the immunoconjugate, the nucleic acid molecule, the vector, the cell and/or the pharmaceutical composition according to claim 61, wherein the tumor includes a tumor with a high expression of PD-1 or PD-L1.

63. The isolated antigen-binding protein, the polypeptide, the immunoconjugate, the nucleic acid molecule, the vector, the cell and/or the pharmaceutical composition according to any one of claims 61 to 62, wherein the tumor includes a solid tumor and/or a non-solid tumor.

64. The isolated antigen-binding protein, the polypeptide, the immunoconjugate, the nucleic acid molecule, the vector, the cell and/or the pharmaceutical composition according to any one of claims 61 to 63, wherein the tumor includes melanoma, lung cancer, renal cancer, esophageal cancer, head and neck cancer, lymphoma, liver cancer and/or gastric cancer.

65. A method of inhibiting the binding of PD-1 protein to PD-L1 protein, comprising administering the isolated antigen-binding protein of any one of claims 1 to 45, the polypeptide of claim 46, the immunoconjugate of claim 47, the nucleic acid molecule of claim 48, the vector of claim 49, the cell of claim 50 and/or the pharmaceutical composition of claim 52.

66. A method of inhibiting the binding of PD-1 protein to PD-L2 protein, comprising administering the isolated antigen-binding protein of any one of claims 1 to 45, the polypeptide of claim 46, the immunoconjugate of claim 47, the nucleic acid molecule of claim 48, the vector of claim 49, the cell of claim 50 and/or the pharmaceutical composition of claim 52.

67. A method for stimulating the secretion of cytokines in immune cells, comprising administering the isolated antigen-binding protein of any one of claims 1 to 45, the polypeptide of claim 46, the immunoconjugate of claim 47, the nucleic acid molecule of claim 48, the vector of claim 49, the cell of claim 50 and/or the pharmaceutical composition of claim 52.

68. A method for detecting the presence and/or content of PD-1 protein, comprising the isolated antigen-binding protein of any one of claims 1 to 45, the polypeptide of claim 46, the immunoconjugate of claim 47, the nucleic acid molecule of claim 48, the vector of claim 49, the cell of claim 50 and/or the pharmaceutical composition of claim 52.

69. A kit comprising the isolated antigen-binding protein of any one of claims 1 to 45, the polypeptide of claim 46, the immunoconjugate of claim 47, the nucleic acid molecule of claim 48, the vector of claim 49, the cell of claim 50 and/or the pharmaceutical composition of claim 52.
